# EUROPEAN PATENT APPLICATION

(11) **EP 2 011 861 A1**
(43) Date of publication of application: **07.01.2009**
(21) Application number: 07741793.9
(22) Date of filing: 17.04.2007
(51) Int. Cl.: C12N 1/21, C12N 9/10, C12N 15/09, C12P 19/32, C12P 19/40, C12R 1/125

(54) **BACTERIUM CAPABLE OF PRODUCING PURINE SUBSTANCE, AND PROCESS FOR PRODUCTION OF PURINE SUBSTANCE**

(30) Priority: 24.04.2006 JP 2006113235
(71) Applicant: Ajinomoto Co., Inc., Tokyo 104-8315 (JP)
(72) Inventor: ASAHARA, Takayuki, Kawasaki-shi, Kanagawa 210-8681 (JP); MATSUNO, Kiyoshi, Kawasaki-shi, Kanagawa 210-8681 (JP); MORI, Yukiko, Kawasaki-shi, Kanagawa 210-8681 (JP)
(74) Representative: Strehl Schübel-Hopf & Partner
(86) International application number: PCT/JP2007/058357
(87) International publication number: WO 2007/125783

(57) **Abstract**

A purine-derived substance is produced by culturing a bacterium belonging to the genus *Bacillus* which has an ability to produce a purine-derived substance and has been modified so that enzymatic activity of transaldolase is decreased in a medium to cause accumulation of a purine-derived substance in the medium or cells, and collecting the purine-derived substance from the medium or cells.

SEQUENCE LISTING

<110>  Ajinomoto Co., Inc.

<120>  Bacterium capable of producing purine substance, and process for
       production of purine substance
<130>  C757-C7064

<150>  JP2006-119324
<151>  2006-04-24

<160>  36

<170>  PatentIn version 3.3

<210>  1
<211>  636
<212>  DNA
<213>  Bacillus subtilis

<220>
<221>  CDS
<222>  (1)..(636)

<400>  1
atg tta ttc ttt gtt gat aca gcc aat atc gat gaa att aga gaa gcg       48
Met Leu Phe Phe Val Asp Thr Ala Asn Ile Asp Glu Ile Arg Glu Ala
1               5                   10                  15
aat gaa tta gga att ctc gcc ggt gta acg acg aat cct agt tta gta       96
Asn Glu Leu Gly Ile Leu Ala Gly Val Thr Thr Asn Pro Ser Leu Val
            20                  25                  30
gca aag gaa gct aac gta tca ttc cac gac cgt ctt cgc gag atc aca      144
Ala Lys Glu Ala Asn Val Ser Phe His Asp Arg Leu Arg Glu Ile Thr
        35                  40                  45
gac gtc gtg aaa ggg tct gta agc gca gag gtt att tct ttg aaa gct      192
Asp Val Val Lys Gly Ser Val Ser Ala Glu Val Ile Ser Leu Lys Ala
    50                  55                  60
gag gaa atg atc gag gaa gga aaa gaa ctg gcg aag atc gct ccg aac      240
Glu Glu Met Ile Glu Glu Gly Lys Glu Leu Ala Lys Ile Ala Pro Asn
65                  70                  75                  80
att acg gtg aaa atc cca atg acg tct gac ggt tta aaa gcg gta aga      288
Ile Thr Val Lys Ile Pro Met Thr Ser Asp Gly Leu Lys Ala Val Arg
                85                  90                  95
gca ctt act ggc tta ggc atc aaa aca aac gtt aca ttg atc ttc aat      336
Ala Leu Thr Gly Leu Gly Ile Lys Thr Asn Val Thr Leu Ile Phe Asn
            100                 105                 110
gcc aac cag gcg ctt ctt gct gcc aga gcg ggg gca aca tat gta tct      384
Ala Asn Gln Ala Leu Leu Ala Ala Arg Ala Gly Ala Thr Tyr Val Ser
        115                 120                 125
cca ttc ctg gga cgt tta gat gac atc ggc cac aac ggg ctt gac ctg      432
Pro Phe Leu Gly Arg Leu Asp Asp Ile Gly His Asn Gly Leu Asp Leu
    130                 135                 140
att tca gaa gtt aaa cag att ttt gac att cac ggc ctt gac acg caa      480
Ile Ser Glu Val Lys Gln Ile Phe Asp Ile His Gly Leu Asp Thr Gln
145                 150                 155                 160
atc att gca gcg tca atc cgc cat ccg cag cac gtg aca gaa gct gct      528
Ile Ile Ala Ala Ser Ile Arg His Pro Gln His Val Thr Glu Ala Ala
                165                 170                 175
ctt aga ggg gct cat atc ggc aca atg ccg ctg aaa gtc att cat gcg      576
Leu Arg Gly Ala His Ile Gly Thr Met Pro Leu Lys Val Ile His Ala
            180                 185                 190
ctc act aaa cac ccg tta aca gac aaa gga atc gaa caa ttc ctg gca      624

Leu Thr Lys His Pro Leu Thr Asp Lys Gly Ile Glu Gln Phe Leu Ala
        195                 200                 205
gac tgg aac aaa 636
Asp Trp Asn Lys
    210

<210> 2
<211> 212
<212> PRT
<213> Bacillus subtilis

<400> 2
Met Leu Phe Phe Val Asp Thr Ala Asn Ile Asp Glu Ile Arg Glu Ala
1               5                   10                  15
Asn Glu Leu Gly Ile Leu Ala Gly Val Thr Thr Asn Pro Ser Leu Val
            20                  25                  30
Ala Lys Glu Ala Asn Val Ser Phe His Asp Arg Leu Arg Glu Ile Thr
        35                  40                  45
Asp Val Val Lys Gly Ser Val Ser Ala Glu Val Ile Ser Leu Lys Ala
    50                  55                  60
Glu Glu Met Ile Glu Glu Gly Lys Glu Leu Ala Lys Ile Ala Pro Asn
65                  70                  75                  80
Ile Thr Val Lys Ile Pro Met Thr Ser Asp Gly Leu Lys Ala Val Arg
                85                  90                  95
Ala Leu Thr Gly Leu Gly Ile Lys Thr Asn Val Thr Leu Ile Phe Asn
            100                 105                 110
Ala Asn Gln Ala Leu Leu Ala Ala Arg Ala Gly Ala Thr Tyr Val Ser
        115                 120                 125
Pro Phe Leu Gly Arg Leu Asp Asp Ile Gly His Asn Gly Leu Asp Leu
    130                 135                 140
Ile Ser Glu Val Lys Gln Ile Phe Asp Ile His Gly Leu Asp Thr Gln
145                 150                 155                 160
Ile Ile Ala Ala Ser Ile Arg His Pro Gln His Val Thr Glu Ala Ala
                165                 170                 175
Leu Arg Gly Ala His Ile Gly Thr Met Pro Leu Lys Val Ile His Ala
            180                 185                 190
Leu Thr Lys His Pro Leu Thr Asp Lys Gly Ile Glu Gln Phe Leu Ala
        195                 200                 205
Asp Trp Asn Lys
    210

<210> 3
<211> 954
<212> DNA
<213> Bacillus subtilis

<220>
<221> CDS
<222> (1)..(954)

<400> 3
atg tct aat caa tac gga gat aag aat tta aag att ttt tct ttg aat 48
Met Ser Asn Gln Tyr Gly Asp Lys Asn Leu Lys Ile Phe Ser Leu Asn
1               5                   10                  15
tcg aat cca gag ctt gca aaa gaa atc gca gat ata gtt gga gtt caa 96
Ser Asn Pro Glu Leu Ala Lys Glu Ile Ala Asp Ile Val Gly Val Gln
            20                  25                  30
tta ggg aaa tgt tct gtc aca aga ttt agt gac ggg gaa gtc caa att 144
Leu Gly Lys Cys Ser Val Thr Arg Phe Ser Asp Gly Glu Val Gln Ile
        35                  40                  45
aat atc gaa gaa agt att cgc gga tgt gat tgt tac atc atc cag tct 192
Asn Ile Glu Glu Ser Ile Arg Gly Cys Asp Cys Tyr Ile Ile Gln Ser
    50                  55                  60

aca agt gac ccc gtt aac gag cat att atg gaa ctg ctg att atg gta 240
Thr Ser Asp Pro Val Asn Glu His Ile Met Glu Leu Leu Ile Met Val
65                  70                  75                  80
gat gcg tta aaa cgc gct tct gca aaa acg att aac att gtt att cct 288
Asp Ala Leu Lys Arg Ala Ser Ala Lys Thr Ile Asn Ile Val Ile Pro
                85                  90                  95
tat tac ggt tat gcg cgt caa gac aga aaa gca aga tcc cgt gag cca 336
Tyr Tyr Gly Tyr Ala Arg Gln Asp Arg Lys Ala Arg Ser Arg Glu Pro
            100                 105                 110
atc aca gct aaa ctt ttc gct aac ctg ctt gaa aca gcc ggt gcg act 384
Ile Thr Ala Lys Leu Phe Ala Asn Leu Leu Glu Thr Ala Gly Ala Thr
        115                 120                 125
cgt gtg att gca ctt gac ctg cat gcg ccg caa att caa gga ttc ttt 432
Arg Val Ile Ala Leu Asp Leu His Ala Pro Gln Ile Gln Gly Phe Phe
    130                 135                 140
gat ata ccg att gac cac tta atg ggt gtt ccg att tta gga gaa tat 480
Asp Ile Pro Ile Asp His Leu Met Gly Val Pro Ile Leu Gly Glu Tyr
145                 150                 155                 160
ttt gaa ggc aaa aat ctt gaa gat atc gtc att gtt tca cca gac cat 528
Phe Glu Gly Lys Asn Leu Glu Asp Ile Val Ile Val Ser Pro Asp His
                165                 170                 175
ggc ggt gtg aca cgt gcc cgc aaa ctg gct gac cga cta aaa gcg cca 576
Gly Gly Val Thr Arg Ala Arg Lys Leu Ala Asp Arg Leu Lys Ala Pro
            180                 185                 190
att gcg att atc gat aaa cgc cgt ccg cgt cca aac gtg gcg gaa gtc 624
Ile Ala Ile Ile Asp Lys Arg Arg Pro Arg Pro Asn Val Ala Glu Val
        195                 200                 205
atg aat att gta ggt aac atc gaa ggg aag act gct atc ctc atc gat 672
Met Asn Ile Val Gly Asn Ile Glu Gly Lys Thr Ala Ile Leu Ile Asp
    210                 215                 220
gac att att gat act gca ggt acg att aca ctt gct gct aat gcg ctc 720
Asp Ile Ile Asp Thr Ala Gly Thr Ile Thr Leu Ala Ala Asn Ala Leu
225                 230                 235                 240
gtt gaa aac gga gcg aaa gaa gta tat gca tgc tgt aca cac cct gta 768
Val Glu Asn Gly Ala Lys Glu Val Tyr Ala Cys Cys Thr His Pro Val
                245                 250                 255
cta tca ggc cct gcg gtt gaa cgg att aat aat tca aca att aaa gag 816
Leu Ser Gly Pro Ala Val Glu Arg Ile Asn Asn Ser Thr Ile Lys Glu
            260                 265                 270
ctt gtt gtg aca aac agc atc aag ctt cct gaa gaa aag aaa att gaa 864
Leu Val Val Thr Asn Ser Ile Lys Leu Pro Glu Glu Lys Lys Ile Glu
        275                 280                 285
cgc ttt aag cag ctt tca gtc gga ccg ctt ctg gcc gaa gcg att att 912
Arg Phe Lys Gln Leu Ser Val Gly Pro Leu Leu Ala Glu Ala Ile Ile
    290                 295                 300
cgc gtt cat gag cag caa tca gtc agc tat ctg ttc agc taa 954
Arg Val His Glu Gln Gln Ser Val Ser Tyr Leu Phe Ser
305                 310                 315

<210> 4
<211> 317
<212> PRT
<213> Bacillus subtilis

<400> 4
Met Ser Asn Gln Tyr Gly Asp Lys Asn Leu Lys Ile Phe Ser Leu Asn
1               5                   10                  15
Ser Asn Pro Glu Leu Ala Lys Glu Ile Ala Asp Ile Val Gly Val Gln
            20                  25                  30
Leu Gly Lys Cys Ser Val Thr Arg Phe Ser Asp Gly Glu Val Gln Ile
        35                  40                  45
Asn Ile Glu Glu Ser Ile Arg Gly Cys Asp Cys Tyr Ile Ile Gln Ser
    50                  55                  60

Thr Ser Asp Pro Val Asn Glu His Ile Met Glu Leu Leu Ile Met Val
65                  70                  75                  80
Asp Ala Leu Lys Arg Ala Ser Ala Lys Thr Ile Asn Ile Val Ile Pro
                85                  90                  95
Tyr Tyr Gly Tyr Ala Arg Gln Asp Arg Lys Ala Arg Ser Arg Glu Pro
            100                 105                 110
Ile Thr Ala Lys Leu Phe Ala Asn Leu Leu Glu Thr Ala Gly Ala Thr
        115                 120                 125
Arg Val Ile Ala Leu Asp Leu His Ala Pro Gln Ile Gln Gly Phe Phe
    130                 135                 140
Asp Ile Pro Ile Asp His Leu Met Gly Val Pro Ile Leu Gly Glu Tyr
145                 150                 155                 160
Phe Glu Gly Lys Asn Leu Glu Asp Ile Val Ile Val Ser Pro Asp His
                165                 170                 175
Gly Gly Val Thr Arg Ala Arg Lys Leu Ala Asp Arg Leu Lys Ala Pro
            180                 185                 190
Ile Ala Ile Ile Asp Lys Arg Arg Pro Arg Pro Asn Val Ala Glu Val
        195                 200                 205
Met Asn Ile Val Gly Asn Ile Glu Gly Lys Thr Ala Ile Leu Ile Asp
    210                 215                 220
Asp Ile Ile Asp Thr Ala Gly Thr Ile Thr Leu Ala Ala Asn Ala Leu
225                 230                 235                 240
Val Glu Asn Gly Ala Lys Glu Val Tyr Ala Cys Cys Thr His Pro Val
                245                 250                 255
Leu Ser Gly Pro Ala Val Glu Arg Ile Asn Asn Ser Thr Ile Lys Glu
            260                 265                 270
Leu Val Val Thr Asn Ser Ile Lys Leu Pro Glu Glu Lys Lys Ile Glu
        275                 280                 285
Arg Phe Lys Gln Leu Ser Val Gly Pro Leu Leu Ala Glu Ala Ile Ile
    290                 295                 300
Arg Val His Glu Gln Gln Ser Val Ser Tyr Leu Phe Ser
305                 310                 315

<210>  5
<211>  858
<212>  DNA
<213>  Bacillus subtilis

<220>
<221>  CDS
<222>  (1)..(858)

<400>  5
atg aag ttt cgt cgc agc ggc aga ttg gtg gac tta aca aat tat ttg       48
Met Lys Phe Arg Arg Ser Gly Arg Leu Val Asp Leu Thr Asn Tyr Leu
1               5                   10                  15
tta acc cat ccg cac gag tta ata ccg cta acc ttt ttc tct gag cgg       96
Leu Thr His Pro His Glu Leu Ile Pro Leu Thr Phe Phe Ser Glu Arg
            20                  25                  30
tat gaa tct gca aaa tca tcg atc agt gaa gat tta aca att att aaa      144
Tyr Glu Ser Ala Lys Ser Ser Ile Ser Glu Asp Leu Thr Ile Ile Lys
        35                  40                  45
caa acc ttt gaa cag cag ggg att ggt act ttg ctt act gtt ccc gga      192
Gln Thr Phe Glu Gln Gln Gly Ile Gly Thr Leu Leu Thr Val Pro Gly
    50                  55                  60
gct gcc gga ggc gtt aaa tat att ccg aaa atg aag cag gct gaa gct      240
Ala Ala Gly Gly Val Lys Tyr Ile Pro Lys Met Lys Gln Ala Glu Ala
65                  70                  75                  80
gaa gag ttt gtg cag aca ctt gga cag tcg ctg gca aat cct gag cgt      288
Glu Glu Phe Val Gln Thr Leu Gly Gln Ser Leu Ala Asn Pro Glu Arg
                85                  90                  95
atc ctt ccg ggc ggt tat gta tat tta acg gat atc tta gga aag cca      336
Ile Leu Pro Gly Gly Tyr Val Tyr Leu Thr Asp Ile Leu Gly Lys Pro

                100                 105                 110
tct gta ctc tcc aag gta ggg aag ctg ttt gct tcc gtg ttt gca gag       384
Ser Val Leu Ser Lys Val Gly Lys Leu Phe Ala Ser Val Phe Ala Glu
        115                 120                 125
cgc gaa att gat gtt gtc atg acc gtt gcc acg aaa ggc atc cct ctt       432
Arg Glu Ile Asp Val Val Met Thr Val Ala Thr Lys Gly Ile Pro Leu
    130                 135                 140
gcg tac gca gct gca agc tat ttg aat gtg cct gtt gtg atc gtt cgt       480
Ala Tyr Ala Ala Ala Ser Tyr Leu Asn Val Pro Val Val Ile Val Arg
145                 150                 155                 160
aaa gac aat aag gta aca gag ggc tcc aca gtc agc att aat tac gtt       528
Lys Asp Asn Lys Val Thr Glu Gly Ser Thr Val Ser Ile Asn Tyr Val
                165                 170                 175
tca ggc tcc tca aac cgc att caa aca atg tca ctt gcg aaa aga agc       576
Ser Gly Ser Ser Asn Arg Ile Gln Thr Met Ser Leu Ala Lys Arg Ser
            180                 185                 190
atg aaa acg ggt tca aac gta ctc att att gat gac ttt atg aaa gca       624
Met Lys Thr Gly Ser Asn Val Leu Ile Ile Asp Asp Phe Met Lys Ala
        195                 200                 205
ggc ggc acc att aat ggt atg att aac ctg ttg gat gag ttt aac gca       672
Gly Gly Thr Ile Asn Gly Met Ile Asn Leu Leu Asp Glu Phe Asn Ala
    210                 215                 220
aat gtg gcg gga atc ggc gtc tta gtt gaa gcc gaa gga gta gat gaa       720
Asn Val Ala Gly Ile Gly Val Leu Val Glu Ala Glu Gly Val Asp Glu
225                 230                 235                 240
cgt ctt gtt gac gaa tat atg tca ctt ctt act ctt tca acc atc aac       768
Arg Leu Val Asp Glu Tyr Met Ser Leu Leu Thr Leu Ser Thr Ile Asn
                245                 250                 255
atg aaa gag aag tcc att gaa att cag aat ggc aat ttt ctg cgt ttt       816
Met Lys Glu Lys Ser Ile Glu Ile Gln Asn Gly Asn Phe Leu Arg Phe
            260                 265                 270
ttt aaa gac aat ctt tta aag aat gga gag aca gaa tca tga               858
Phe Lys Asp Asn Leu Leu Lys Asn Gly Glu Thr Glu Ser
        275                 280                 285

<210>  6
<211>  285
<212>  PRT
<213>  Bacillus subtilis

<400>  6
Met Lys Phe Arg Arg Ser Gly Arg Leu Val Asp Leu Thr Asn Tyr Leu
1               5                   10                  15
Leu Thr His Pro His Glu Leu Ile Pro Leu Thr Phe Phe Ser Glu Arg
            20                  25                  30
Tyr Glu Ser Ala Lys Ser Ser Ile Ser Glu Asp Leu Thr Ile Ile Lys
        35                  40                  45
Gln Thr Phe Glu Gln Gln Gly Ile Gly Thr Leu Leu Thr Val Pro Gly
    50                  55                  60
Ala Ala Gly Gly Val Lys Tyr Ile Pro Lys Met Lys Gln Ala Glu Ala
65                  70                  75                  80
Glu Glu Phe Val Gln Thr Leu Gly Gln Ser Leu Ala Asn Pro Glu Arg
                85                  90                  95
Ile Leu Pro Gly Gly Tyr Val Tyr Leu Thr Asp Ile Leu Gly Lys Pro
            100                 105                 110
Ser Val Leu Ser Lys Val Gly Lys Leu Phe Ala Ser Val Phe Ala Glu
        115                 120                 125
Arg Glu Ile Asp Val Val Met Thr Val Ala Thr Lys Gly Ile Pro Leu
    130                 135                 140
Ala Tyr Ala Ala Ala Ser Tyr Leu Asn Val Pro Val Val Ile Val Arg
145                 150                 155                 160
Lys Asp Asn Lys Val Thr Glu Gly Ser Thr Val Ser Ile Asn Tyr Val
                165                 170                 175

Ser Gly Ser Ser Asn Arg Ile Gln Thr Met Ser Leu Ala Lys Arg Ser
            180                 185                 190
Met Lys Thr Gly Ser Asn Val Leu Ile Ile Asp Asp Phe Met Lys Ala
        195                 200                 205
Gly Gly Thr Ile Asn Gly Met Ile Asn Leu Leu Asp Glu Phe Asn Ala
    210                 215                 220
Asn Val Ala Gly Ile Gly Val Leu Val Glu Ala Glu Gly Val Asp Glu
225                 230                 235                 240
Arg Leu Val Asp Glu Tyr Met Ser Leu Leu Thr Leu Ser Thr Ile Asn
                245                 250                 255
Met Lys Glu Lys Ser Ile Glu Ile Gln Asn Gly Asn Phe Leu Arg Phe
            260                 265                 270
Phe Lys Asp Asn Leu Leu Lys Asn Gly Glu Thr Glu Ser
        275                 280                 285

<210>  7
<211>  702
<212>  DNA
<213>  Bacillus subtilis

<220>
<221>  CDS
<222>  (1)..(702)

<400>  7
atg agt gta cat ata ggt gct gaa aaa gga caa att gcg gat act gtg      48
Met Ser Val His Ile Gly Ala Glu Lys Gly Gln Ile Ala Asp Thr Val
1               5                   10                  15
ctt ttg ccg gga gat cct ctc aga gca aaa ttt att gca gaa acg tat      96
Leu Leu Pro Gly Asp Pro Leu Arg Ala Lys Phe Ile Ala Glu Thr Tyr
            20                  25                  30
ctt gaa aat gta gaa tgc tac aat gaa gtc aga ggc atg tat gga ttt      144
Leu Glu Asn Val Glu Cys Tyr Asn Glu Val Arg Gly Met Tyr Gly Phe
        35                  40                  45
acg ggt aca tat aaa ggt aaa aaa atc tca gta caa ggc acg gga atg      192
Thr Gly Thr Tyr Lys Gly Lys Lys Ile Ser Val Gln Gly Thr Gly Met
    50                  55                  60
gga gtt ccg tct att tca att tat gtg aat gaa tta att caa agc tac      240
Gly Val Pro Ser Ile Ser Ile Tyr Val Asn Glu Leu Ile Gln Ser Tyr
65                  70                  75                  80
gat gtg caa aat cta ata aga gtc ggt tcc tgc ggc gct att cgt aaa      288
Asp Val Gln Asn Leu Ile Arg Val Gly Ser Cys Gly Ala Ile Arg Lys
                85                  90                  95
gat gtc aaa gtg cga gac gtc att ttg gcg atg acc tcc tca act gat      336
Asp Val Lys Val Arg Asp Val Ile Leu Ala Met Thr Ser Ser Thr Asp
            100                 105                 110
tca caa atg aac aga gtt gct ttc gga agc gtt gat ttt gcg cct tgc      384
Ser Gln Met Asn Arg Val Ala Phe Gly Ser Val Asp Phe Ala Pro Cys
        115                 120                 125
gca gat ttc gag ctt tta aaa aat gcc tat gat gcc gca aag gat aaa      432
Ala Asp Phe Glu Leu Leu Lys Asn Ala Tyr Asp Ala Ala Lys Asp Lys
    130                 135                 140
ggt gtg ccg gtg act gta gga agc gta ttt aca gct gac cag ttc tac      480
Gly Val Pro Val Thr Val Gly Ser Val Phe Thr Ala Asp Gln Phe Tyr
145                 150                 155                 160
aat gac gat tcg caa att gaa aaa ctt gca aaa tac ggt gtg ctt ggc      528
Asn Asp Asp Ser Gln Ile Glu Lys Leu Ala Lys Tyr Gly Val Leu Gly
                165                 170                 175
gtt gaa atg gaa aca act gca ttg tat aca tta gca gcg aag cac gga      576
Val Glu Met Glu Thr Thr Ala Leu Tyr Thr Leu Ala Ala Lys His Gly
            180                 185                 190
aga aaa gcc ctg tca att ctc acc gtg agt gat cac gta tta aca gga      624
Arg Lys Ala Leu Ser Ile Leu Thr Val Ser Asp His Val Leu Thr Gly

195                 200                 205
gaa gaa acg aca gcg gaa gag cgt caa acg aca ttt cat gat atg ata      672
Glu Glu Thr Thr Ala Glu Glu Arg Gln Thr Thr Phe His Asp Met Ile
    210                 215                 220
gaa gtg gct tta cat tcc gta tca caa taa                              702
Glu Val Ala Leu His Ser Val Ser Gln
225                 230

<210>  8
<211>  233
<212>  PRT
<213>  Bacillus subtilis

<400>  8
Met Ser Val His Ile Gly Ala Glu Lys Gly Gln Ile Ala Asp Thr Val
1               5                   10                  15
Leu Leu Pro Gly Asp Pro Leu Arg Ala Lys Phe Ile Ala Glu Thr Tyr
            20                  25                  30
Leu Glu Asn Val Glu Cys Tyr Asn Glu Val Arg Gly Met Tyr Gly Phe
        35                  40                  45
Thr Gly Thr Tyr Lys Gly Lys Lys Ile Ser Val Gln Gly Thr Gly Met
    50                  55                  60
Gly Val Pro Ser Ile Ser Ile Tyr Val Asn Glu Leu Ile Gln Ser Tyr
65                  70                  75                  80
Asp Val Gln Asn Leu Ile Arg Val Gly Ser Cys Gly Ala Ile Arg Lys
                85                  90                  95
Asp Val Lys Val Arg Asp Val Ile Leu Ala Met Thr Ser Ser Thr Asp
            100                 105                 110
Ser Gln Met Asn Arg Val Ala Phe Gly Ser Val Asp Phe Ala Pro Cys
        115                 120                 125
Ala Asp Phe Glu Leu Leu Lys Asn Ala Tyr Asp Ala Ala Lys Asp Lys
    130                 135                 140
Gly Val Pro Val Thr Val Gly Ser Val Phe Thr Ala Asp Gln Phe Tyr
145                 150                 155                 160
Asn Asp Asp Ser Gln Ile Glu Lys Leu Ala Lys Tyr Gly Val Leu Gly
                165                 170                 175
Val Glu Met Glu Thr Thr Ala Leu Tyr Thr Leu Ala Ala Lys His Gly
            180                 185                 190
Arg Lys Ala Leu Ser Ile Leu Thr Val Ser Asp His Val Leu Thr Gly
        195                 200                 205
Glu Glu Thr Thr Ala Glu Glu Arg Gln Thr Thr Phe His Asp Met Ile
    210                 215                 220
Glu Val Ala Leu His Ser Val Ser Gln
225                 230

<210>  9
<211>  816
<212>  DNA
<213>  Bacillus subtilis

<220>
<221>  CDS
<222>  (1)..(816)

<400>  9
ttg aag gac aga att gaa cgc gca gcc gct ttt att aaa caa aac ctg       48
Leu Lys Asp Arg Ile Glu Arg Ala Ala Ala Phe Ile Lys Gln Asn Leu
1               5                   10                  15
ccg gaa tct cca aag atc ggc ctt att tta ggc tca ggt ctt ggc att       96
Pro Glu Ser Pro Lys Ile Gly Leu Ile Leu Gly Ser Gly Leu Gly Ile
            20                  25                  30
ttg gcg gac gaa atc gaa aat ccg gtc aag ctg aaa tat gaa gat ata      144
Leu Ala Asp Glu Ile Glu Asn Pro Val Lys Leu Lys Tyr Glu Asp Ile

        35                  40                  45
cct gaa ttc ccg gta tct act gtt gaa ggg cat gcc gga cag ctt gtg 192
Pro Glu Phe Pro Val Ser Thr Val Glu Gly His Ala Gly Gln Leu Val
    50                  55                  60
ctt ggc act ctt gaa gga gtt tcc gtc att gca atg cag ggc cgc ttt 240
Leu Gly Thr Leu Glu Gly Val Ser Val Ile Ala Met Gln Gly Arg Phe
65                  70                  75                  80
cat ttt tat gaa ggc tac tca atg gag aaa gtc aca ttc cct gta cgc 288
His Phe Tyr Glu Gly Tyr Ser Met Glu Lys Val Thr Phe Pro Val Arg
                85                  90                  95
gtg atg aaa gcg ctc ggt gtg gaa gcg ttg atc gtg aca aat gcc gca 336
Val Met Lys Ala Leu Gly Val Glu Ala Leu Ile Val Thr Asn Ala Ala
            100                 105                 110
ggc ggt gtc aac act gaa ttc cgt gcg gga gat tta atg att att acc 384
Gly Gly Val Asn Thr Glu Phe Arg Ala Gly Asp Leu Met Ile Ile Thr
        115                 120                 125
gat cat atc aac ttt atg gga aca aac ccg tta atc ggg cca aac gaa 432
Asp His Ile Asn Phe Met Gly Thr Asn Pro Leu Ile Gly Pro Asn Glu
    130                 135                 140
gca gat ttc ggc gcc aga ttt cca gat atg tct tca gcc tat gac aaa 480
Ala Asp Phe Gly Ala Arg Phe Pro Asp Met Ser Ser Ala Tyr Asp Lys
145                 150                 155                 160
gat ctg tcc agc ctg gct gaa aag att gcg aaa gac ctt aat atc cca 528
Asp Leu Ser Ser Leu Ala Glu Lys Ile Ala Lys Asp Leu Asn Ile Pro
                165                 170                 175
att caa aaa ggc gtg tac act gct gtg aca gga cct tct tac gaa aca 576
Ile Gln Lys Gly Val Tyr Thr Ala Val Thr Gly Pro Ser Tyr Glu Thr
            180                 185                 190
ccg gca gaa gtc cgt ttc tta aga acg atg ggc tct gat gca gtc ggc 624
Pro Ala Glu Val Arg Phe Leu Arg Thr Met Gly Ser Asp Ala Val Gly
        195                 200                 205
atg tct act gtt ccg gaa gtc att gta gcg aat cat gcg gga atg cgg 672
Met Ser Thr Val Pro Glu Val Ile Val Ala Asn His Ala Gly Met Arg
    210                 215                 220
gtt ctt ggc att tcc tgc atc tct aac gcg gca gcc gga att ctg gat 720
Val Leu Gly Ile Ser Cys Ile Ser Asn Ala Ala Ala Gly Ile Leu Asp
225                 230                 235                 240
cag cct tta agt cac gat gaa gtt atg gaa gtg acc gaa aaa gta aaa 768
Gln Pro Leu Ser His Asp Glu Val Met Glu Val Thr Glu Lys Val Lys
                245                 250                 255
gct gga ttc tta aag ctt gtt aaa gcg atc gtc gct cag tac gaa taa 816
Ala Gly Phe Leu Lys Leu Val Lys Ala Ile Val Ala Gln Tyr Glu
            260                 265                 270

<210> 10
<211> 271
<212> PRT
<213> Bacillus subtilis

<400> 10
Leu Lys Asp Arg Ile Glu Arg Ala Ala Ala Phe Ile Lys Gln Asn Leu
1               5                   10                  15
Pro Glu Ser Pro Lys Ile Gly Leu Ile Leu Gly Ser Gly Leu Gly Ile
            20                  25                  30
Leu Ala Asp Glu Ile Glu Asn Pro Val Lys Leu Lys Tyr Glu Asp Ile
        35                  40                  45
Pro Glu Phe Pro Val Ser Thr Val Glu Gly His Ala Gly Gln Leu Val
    50                  55                  60
Leu Gly Thr Leu Glu Gly Val Ser Val Ile Ala Met Gln Gly Arg Phe
65                  70                  75                  80
His Phe Tyr Glu Gly Tyr Ser Met Glu Lys Val Thr Phe Pro Val Arg
                85                  90                  95
Val Met Lys Ala Leu Gly Val Glu Ala Leu Ile Val Thr Asn Ala Ala

                100                 105                 110
Gly Gly Val Asn Thr Glu Phe Arg Ala Gly Asp Leu Met Ile Ile Thr
        115                 120                 125
Asp His Ile Asn Phe Met Gly Thr Asn Pro Leu Ile Gly Pro Asn Glu
    130                 135                 140
Ala Asp Phe Gly Ala Arg Phe Pro Asp Met Ser Ser Ala Tyr Asp Lys
145                 150                 155                 160
Asp Leu Ser Ser Leu Ala Glu Lys Ile Ala Lys Asp Leu Asn Ile Pro
                165                 170                 175
Ile Gln Lys Gly Val Tyr Thr Ala Val Thr Gly Pro Ser Tyr Glu Thr
            180                 185                 190
Pro Ala Glu Val Arg Phe Leu Arg Thr Met Gly Ser Asp Ala Val Gly
        195                 200                 205
Met Ser Thr Val Pro Glu Val Ile Val Ala Asn His Ala Gly Met Arg
    210                 215                 220
Val Leu Gly Ile Ser Cys Ile Ser Asn Ala Ala Ala Gly Ile Leu Asp
225                 230                 235                 240
Gln Pro Leu Ser His Asp Glu Val Met Glu Val Thr Glu Lys Val Lys
                245                 250                 255
Ala Gly Phe Leu Lys Leu Val Lys Ala Ile Val Ala Gln Tyr Glu
            260                 265                 270

<210>  11
<211>  1293
<212>  DNA
<213>  Bacillus subtilis

<220>
<221>  CDS
<222>  (1)..(1293)

<400>  11
atg tct tca gta gtt gta gta ggt acg caa tgg ggc gat gaa gga aaa       48
Met Ser Ser Val Val Val Val Gly Thr Gln Trp Gly Asp Glu Gly Lys
1               5                   10                  15
ggt aaa att aca gat ttc cta tca gaa aat gca gaa gtg atc gcc cgt       96
Gly Lys Ile Thr Asp Phe Leu Ser Glu Asn Ala Glu Val Ile Ala Arg
            20                  25                  30
tat caa ggc gga aat aac gca ggg cat aca atc aag ttt gac gga atc      144
Tyr Gln Gly Gly Asn Asn Ala Gly His Thr Ile Lys Phe Asp Gly Ile
        35                  40                  45
aca tat aag ctt cac tta atc ccg tct gga att ttc tat aag gat aaa      192
Thr Tyr Lys Leu His Leu Ile Pro Ser Gly Ile Phe Tyr Lys Asp Lys
    50                  55                  60
acg tgt gta atc gga aac gga atg gtt gta gat ccg aaa gca tta gtc      240
Thr Cys Val Ile Gly Asn Gly Met Val Val Asp Pro Lys Ala Leu Val
65                  70                  75                  80
aca gag ctt gcg tat ctt cat gag cgc aac gtg agt aca gat aac ctg      288
Thr Glu Leu Ala Tyr Leu His Glu Arg Asn Val Ser Thr Asp Asn Leu
                85                  90                  95
aga atc agc aac aga gct cac gtc att ctg ccg tat cat ttg aaa ttg      336
Arg Ile Ser Asn Arg Ala His Val Ile Leu Pro Tyr His Leu Lys Leu
            100                 105                 110
gat gaa gtg gaa gaa gag cgt aaa ggg gct aac aag atc ggc aca acg      384
Asp Glu Val Glu Glu Glu Arg Lys Gly Ala Asn Lys Ile Gly Thr Thr
        115                 120                 125
aaa aaa gga atc ggc cct gct tac atg gat aaa gca gcc cgc atc gga      432
Lys Lys Gly Ile Gly Pro Ala Tyr Met Asp Lys Ala Ala Arg Ile Gly
    130                 135                 140
att cgc atc gcg gat ctg tta gac cgt gac gcg ttt gcg gaa aag ctt      480
Ile Arg Ile Ala Asp Leu Leu Asp Arg Asp Ala Phe Ala Glu Lys Leu
145                 150                 155                 160
gag cgc aat ctt gaa gaa aaa aac cgt ctt ctc gag aaa atg tac gag      528

Glu Arg Asn Leu Glu Glu Lys Asn Arg Leu Leu Glu Lys Met Tyr Glu
                165                 170                 175
aca gaa ggg ttt aaa ctt gag gat atc tta gac gaa tat tat gag tac      576
Thr Glu Gly Phe Lys Leu Glu Asp Ile Leu Asp Glu Tyr Tyr Glu Tyr
            180                 185                 190
gga cag cag att aaa aag tat gtt tgc gat aca tct gtt gtc tta aac      624
Gly Gln Gln Ile Lys Lys Tyr Val Cys Asp Thr Ser Val Val Leu Asn
        195                 200                 205
gat gct ctt gat gaa ggg cgc cgt gta tta ttt gaa ggc gca caa ggg      672
Asp Ala Leu Asp Glu Gly Arg Arg Val Leu Phe Glu Gly Ala Gln Gly
    210                 215                 220
gtt atg ctc gat atc gac caa gga aca tac ccg ttt gtt acg tca tct      720
Val Met Leu Asp Ile Asp Gln Gly Thr Tyr Pro Phe Val Thr Ser Ser
225                 230                 235                 240
aac ccg gtt gcc ggc ggt gtc acg atc ggt tct ggt gtc ggc ccg acc      768
Asn Pro Val Ala Gly Gly Val Thr Ile Gly Ser Gly Val Gly Pro Thr
                245                 250                 255
aaa atc aag cac gtt gtc ggt gta tca aaa gca tat acg act cgt gtc      816
Lys Ile Lys His Val Val Gly Val Ser Lys Ala Tyr Thr Thr Arg Val
            260                 265                 270
ggc gac ggt cct ttt ccg act gag ctg aaa gat gaa atc ggc gat caa      864
Gly Asp Gly Pro Phe Pro Thr Glu Leu Lys Asp Glu Ile Gly Asp Gln
        275                 280                 285
atc cgt gaa gtc gga cgc gaa tat gga aca aca aca ggc cgc ccg cgc      912
Ile Arg Glu Val Gly Arg Glu Tyr Gly Thr Thr Thr Gly Arg Pro Arg
    290                 295                 300
cgt gtc ggc tgg ttt gac agc gtt gtt gtc cgc cac gcc cgc cgt gtg      960
Arg Val Gly Trp Phe Asp Ser Val Val Val Arg His Ala Arg Arg Val
305                 310                 315                 320
agc gga att aca gat ctt tct ctg aac tca att gac gtc cta gca gga     1008
Ser Gly Ile Thr Asp Leu Ser Leu Asn Ser Ile Asp Val Leu Ala Gly
                325                 330                 335
att gaa acg ttg aaa atc tgt gtg gcg tac cgc tac aaa ggc gaa atc     1056
Ile Glu Thr Leu Lys Ile Cys Val Ala Tyr Arg Tyr Lys Gly Glu Ile
            340                 345                 350
att gaa gaa ttc cca gca agt ctt aag gca ctt gct gaa tgt gag ccg     1104
Ile Glu Glu Phe Pro Ala Ser Leu Lys Ala Leu Ala Glu Cys Glu Pro
        355                 360                 365
gta tat gaa gaa atg ccg ggc tgg act gag gat att aca ggt gcg aag     1152
Val Tyr Glu Glu Met Pro Gly Trp Thr Glu Asp Ile Thr Gly Ala Lys
    370                 375                 380
agc ttg agc gag ctt ccg gaa aat gcg cgc cat tat ctt gag cgt gtg     1200
Ser Leu Ser Glu Leu Pro Glu Asn Ala Arg His Tyr Leu Glu Arg Val
385                 390                 395                 400
tct cag ctg aca ggc att ccg ctt tct att ttc tct gtc ggt cca gac     1248
Ser Gln Leu Thr Gly Ile Pro Leu Ser Ile Phe Ser Val Gly Pro Asp
                405                 410                 415
cgc tca caa aca aat gtc ctt cgc agt gtg tac cgt gcg aac taa         1293
Arg Ser Gln Thr Asn Val Leu Arg Ser Val Tyr Arg Ala Asn
            420                 425                 430
<210>  12
<211>  430
<212>  PRT
<213>  Bacillus subtilis

<400>  12
Met Ser Ser Val Val Val Val Gly Thr Gln Trp Gly Asp Glu Gly Lys
1               5                   10                  15
Gly Lys Ile Thr Asp Phe Leu Ser Glu Asn Ala Glu Val Ile Ala Arg
            20                  25                  30
Tyr Gln Gly Gly Asn Asn Ala Gly His Thr Ile Lys Phe Asp Gly Ile
        35                  40                  45
Thr Tyr Lys Leu His Leu Ile Pro Ser Gly Ile Phe Tyr Lys Asp Lys

50                  55                  60
Thr Cys Val Ile Gly Asn Gly Met Val Val Asp Pro Lys Ala Leu Val
65                  70                  75                  80
Thr Glu Leu Ala Tyr Leu His Glu Arg Asn Val Ser Thr Asp Asn Leu
                85                  90                  95
Arg Ile Ser Asn Arg Ala His Val Ile Leu Pro Tyr His Leu Lys Leu
            100                 105                 110
Asp Glu Val Glu Glu Glu Arg Lys Gly Ala Asn Lys Ile Gly Thr Thr
        115                 120                 125
Lys Lys Gly Ile Gly Pro Ala Tyr Met Asp Lys Ala Ala Arg Ile Gly
    130                 135                 140
Ile Arg Ile Ala Asp Leu Leu Asp Arg Asp Ala Phe Ala Glu Lys Leu
145                 150                 155                 160
Glu Arg Asn Leu Glu Glu Lys Asn Arg Leu Leu Glu Lys Met Tyr Glu
                165                 170                 175
Thr Glu Gly Phe Lys Leu Glu Asp Ile Leu Asp Glu Tyr Tyr Glu Tyr
            180                 185                 190
Gly Gln Gln Ile Lys Lys Tyr Val Cys Asp Thr Ser Val Val Leu Asn
        195                 200                 205
Asp Ala Leu Asp Glu Gly Arg Arg Val Leu Phe Glu Gly Ala Gln Gly
    210                 215                 220
Val Met Leu Asp Ile Asp Gln Gly Thr Tyr Pro Phe Val Thr Ser Ser
225                 230                 235                 240
Asn Pro Val Ala Gly Gly Val Thr Ile Gly Ser Gly Val Gly Pro Thr
                245                 250                 255
Lys Ile Lys His Val Val Gly Val Ser Lys Ala Tyr Thr Thr Arg Val
            260                 265                 270
Gly Asp Gly Pro Phe Pro Thr Glu Leu Lys Asp Glu Ile Gly Asp Gln
        275                 280                 285
Ile Arg Glu Val Gly Arg Glu Tyr Gly Thr Thr Thr Gly Arg Pro Arg
    290                 295                 300
Arg Val Gly Trp Phe Asp Ser Val Val Val Arg His Ala Arg Arg Val
305                 310                 315                 320
Ser Gly Ile Thr Asp Leu Ser Leu Asn Ser Ile Asp Val Leu Ala Gly
                325                 330                 335
Ile Glu Thr Leu Lys Ile Cys Val Ala Tyr Arg Tyr Lys Gly Glu Ile
            340                 345                 350
Ile Glu Glu Phe Pro Ala Ser Leu Lys Ala Leu Ala Glu Cys Glu Pro
        355                 360                 365
Val Tyr Glu Glu Met Pro Gly Trp Thr Glu Asp Ile Thr Gly Ala Lys
    370                 375                 380
Ser Leu Ser Glu Leu Pro Glu Asn Ala Arg His Tyr Leu Glu Arg Val
385                 390                 395                 400
Ser Gln Leu Thr Gly Ile Pro Leu Ser Ile Phe Ser Val Gly Pro Asp
                405                 410                 415
Arg Ser Gln Thr Asn Val Leu Arg Ser Val Tyr Arg Ala Asn
            420                 425                 430
<210>  13
<211>  1542
<212>  DNA
<213>  Bacillus subtilis

<220>
<221>  CDS
<222>  (1)..(1542)

<400>  13
atg tgg gaa agt aaa ttt tca aaa gaa ggc tta acg ttc gac gat gtg        48
Met Trp Glu Ser Lys Phe Ser Lys Glu Gly Leu Thr Phe Asp Asp Val
1               5                   10                  15
ctg ctt gtg cca gca aag tct gag gta ctt ccg cat gat gtg gat tta        96
Leu Leu Val Pro Ala Lys Ser Glu Val Leu Pro His Asp Val Asp Leu
            20                  25                  30

tct gta gaa ctt aca aaa acg tta aag cta aat att cct gtc atc agc 144
Ser Val Glu Leu Thr Lys Thr Leu Lys Leu Asn Ile Pro Val Ile Ser
35 . 40 45
gca ggt atg gac act gta aca gaa tca gca atg gca att gca atg gca 192
Ala Gly Met Asp Thr Val Thr Glu Ser Ala Met Ala Ile Ala Met Ala
50 55 60
aga cag ggc ggc ttg ggc atc att cac aaa aat atg tcc att gaa cag 240
Arg Gln Gly Gly Leu Gly Ile Ile His Lys Asn Met Ser Ile Glu Gln
65 70 75 80
cag gct gaa caa gtt gat aaa gta aag cgt tct gag cgc ggc gtt atc 288
Gln Ala Glu Gln Val Asp Lys Val Lys Arg Ser Glu Arg Gly Val Ile
85 90 95
aca aat ccc ttc ttt tta act cct gat cac caa gta ttt gat gcg gag 336
Thr Asn Pro Phe Phe Leu Thr Pro Asp His Gln Val Phe Asp Ala Glu
100 105 110
cat ttg atg ggg aaa tac aga att tcc ggt gtt ccg att gta aat aac 384
His Leu Met Gly Lys Tyr Arg Ile Ser Gly Val Pro Ile Val Asn Asn
115 120 125
gaa gaa gac cag aag ctt gtt gga att att aca aac cgt gac ctt cgt 432
Glu Glu Asp Gln Lys Leu Val Gly Ile Ile Thr Asn Arg Asp Leu Arg
130 135 140
ttt att tct gac tac tca atg aaa atc agc gac gtc atg acg aaa gaa 480
Phe Ile Ser Asp Tyr Ser Met Lys Ile Ser Asp Val Met Thr Lys Glu
145 150 155 160
gag cta gtt act gca tct gta gga act act ctg gat gaa gct gaa aag 528
Glu Leu Val Thr Ala Ser Val Gly Thr Thr Leu Asp Glu Ala Glu Lys
165 170 175
att ttg caa aaa cat aaa att gaa aag ctt cct ctc gta gat gac cag 576
Ile Leu Gln Lys His Lys Ile Glu Lys Leu Pro Leu Val Asp Asp Gln
180 185 190
aat aaa tta aaa ggt ctt atc aca att aaa gac att gaa aaa gtc att 624
Asn Lys Leu Lys Gly Leu Ile Thr Ile Lys Asp Ile Glu Lys Val Ile
195 200 205
gag ttc ccg aac tca tct aaa gac att cac ggc cgc ctg atc gtt ggc 672
Glu Phe Pro Asn Ser Ser Lys Asp Ile His Gly Arg Leu Ile Val Gly
210 215 220
gcg gca gtt ggt gta act ggc gat aca atg act cgc gtc aaa aag ctt 720
Ala Ala Val Gly Val Thr Gly Asp Thr Met Thr Arg Val Lys Lys Leu
225 230 235 240
gtt gaa gcc aat gtt gat gtg att gtt atc gat aca gct cac gga cac 768
Val Glu Ala Asn Val Asp Val Ile Val Ile Asp Thr Ala His Gly His
245 250 255
tct caa ggc gtt tta aac aca gtt aca aaa atc cgt gaa acg tat ccc 816
Ser Gln Gly Val Leu Asn Thr Val Thr Lys Ile Arg Glu Thr Tyr Pro
260 265 270
gaa tta aac att att gct gga aac gtg gca aca gct gaa gcg aca aga 864
Glu Leu Asn Ile Ile Ala Gly Asn Val Ala Thr Ala Glu Ala Thr Arg
275 280 285
gcg ctt atc gaa gct gga gca gac gtt gtc aaa gtt gga ata ggg cct 912
Ala Leu Ile Glu Ala Gly Ala Asp Val Val Lys Val Gly Ile Gly Pro
290 295 300
ggt tca att tgt act aca cgt gtt gta gcc ggg gtg ggt gtt ccg caa 960
Gly Ser Ile Cys Thr Thr Arg Val Val Ala Gly Val Gly Val Pro Gln
305 310 315 320
att aca gca att tat gat tgt gcg act gaa gca aga aaa cac ggc aaa 1008
Ile Thr Ala Ile Tyr Asp Cys Ala Thr Glu Ala Arg Lys His Gly Lys
325 330 335
aca atc atc gcc gac ggt ggg att aaa ttc tct ggc gat atc act aaa 1056
Thr Ile Ile Ala Asp Gly Gly Ile Lys Phe Ser Gly Asp Ile Thr Lys
340 345 350
gca ttg gca gcc ggc gga cat gct gtt atg ctc gga agc ttg ctt gca 1104
Ala Leu Ala Ala Gly Gly His Ala Val Met Leu Gly Ser Leu Leu Ala
355 360 365

ggc aca tca gaa agc cct ggt gaa act gaa atc tac caa ggc aga aga     1152
Gly Thr Ser Glu Ser Pro Gly Glu Thr Glu Ile Tyr Gln Gly Arg Arg
    370                 375                 380
ttt aag gta tac cgc ggc atg gga tca gtt gct gca atg gaa aaa gga     1200
Phe Lys Val Tyr Arg Gly Met Gly Ser Val Ala Ala Met Glu Lys Gly
385                 390                 395                 400
agt aaa gac cgt tac ttc caa gaa gaa aac aaa aaa ttt gtt cct gaa     1248
Ser Lys Asp Arg Tyr Phe Gln Glu Glu Asn Lys Lys Phe Val Pro Glu
                405                 410                 415
gga att gaa gga cgc aca cct tac aaa ggg cca gtt gaa gaa acc gtt     1296
Gly Ile Glu Gly Arg Thr Pro Tyr Lys Gly Pro Val Glu Glu Thr Val
            420                 425                 430
tat cag cta gtc gga ggc ctt cgt tct ggt atg ggg tat tgc ggg tcc     1344
Tyr Gln Leu Val Gly Gly Leu Arg Ser Gly Met Gly Tyr Cys Gly Ser
        435                 440                 445
aaa gat ctg cgt gcg cta aga gaa gaa gct cag ttc att cgc atg act     1392
Lys Asp Leu Arg Ala Leu Arg Glu Glu Ala Gln Phe Ile Arg Met Thr
    450                 455                 460
ggc gca gga ctt cgc gaa agc cat ccg cat gac gta cag att aca gtg     1440
Gly Ala Gly Leu Arg Glu Ser His Pro His Asp Val Gln Ile Thr Val
465                 470                 475                 480
cat cgt aat aag gcg ctt cct ggt cta ttt ggt tct cat cag aaa aaa     1488
His Arg Asn Lys Ala Leu Pro Gly Leu Phe Gly Ser His Gln Lys Lys
                485                 490                 495
aca gga ttt gtg tat gat gaa tgt tgt caa tcc ggc ttt ttt tca tcg     1536
Thr Gly Phe Val Tyr Asp Glu Cys Cys Gln Ser Gly Phe Phe Ser Ser
            500                 505                 510
gat tga                                                             1542
Asp

<210>  14
<211>  513
<212>  PRT
<213>  Bacillus subtilis

<400>  14
Met Trp Glu Ser Lys Phe Ser Lys Glu Gly Leu Thr Phe Asp Asp Val
1               5                   10                  15
Leu Leu Val Pro Ala Lys Ser Glu Val Leu Pro His Asp Val Asp Leu
            20                  25                  30
Ser Val Glu Leu Thr Lys Thr Leu Lys Leu Asn Ile Pro Val Ile Ser
        35                  40                  45
Ala Gly Met Asp Thr Val Thr Glu Ser Ala Met Ala Ile Ala Met Ala
    50                  55                  60
Arg Gln Gly Gly Leu Gly Ile Ile His Lys Asn Met Ser Ile Glu Gln
65                  70                  75                  80
Gln Ala Glu Gln Val Asp Lys Val Lys Arg Ser Glu Arg Gly Val Ile
                85                  90                  95
Thr Asn Pro Phe Phe Leu Thr Pro Asp His Gln Val Phe Asp Ala Glu
            100                 105                 110
His Leu Met Gly Lys Tyr Arg Ile Ser Gly Val Pro Ile Val Asn Asn
        115                 120                 125
Glu Glu Asp Gln Lys Leu Val Gly Ile Ile Thr Asn Arg Asp Leu Arg
    130                 135                 140
Phe Ile Ser Asp Tyr Ser Met Lys Ile Ser Asp Val Met Thr Lys Glu
145                 150                 155                 160
Glu Leu Val Thr Ala Ser Val Gly Thr Thr Leu Asp Glu Ala Glu Lys
                165                 170                 175
Ile Leu Gln Lys His Lys Ile Glu Lys Leu Pro Leu Val Asp Asp Gln
            180                 185                 190
Asn Lys Leu Lys Gly Leu Ile Thr Ile Lys Asp Ile Glu Lys Val Ile
        195                 200                 205
Glu Phe Pro Asn Ser Ser Lys Asp Ile His Gly Arg Leu Ile Val Gly

210                 215                 220
Ala Ala Val Gly Val Thr Gly Asp Thr Met Thr Arg Val Lys Lys Leu
225                 230                 235                 240
Val Glu Ala Asn Val Asp Val Ile Val Ile Asp Thr Ala His Gly His
                245                 250                 255
Ser Gln Gly Val Leu Asn Thr Val Thr Lys Ile Arg Glu Thr Tyr Pro
            260                 265                 270
Glu Leu Asn Ile Ile Ala Gly Asn Val Ala Thr Ala Glu Ala Thr Arg
        275                 280                 285
Ala Leu Ile Glu Ala Gly Ala Asp Val Val Lys Val Gly Ile Gly Pro
    290                 295                 300
Gly Ser Ile Cys Thr Thr Arg Val Val Ala Gly Val Gly Val Pro Gln
305                 310                 315                 320
Ile Thr Ala Ile Tyr Asp Cys Ala Thr Glu Ala Arg Lys His Gly Lys
                325                 330                 335
Thr Ile Ile Ala Asp Gly Gly Ile Lys Phe Ser Gly Asp Ile Thr Lys
            340                 345                 350
Ala Leu Ala Ala Gly Gly His Ala Val Met Leu Gly Ser Leu Leu Ala
        355                 360                 365
Gly Thr Ser Glu Ser Pro Gly Glu Thr Glu Ile Tyr Gln Gly Arg Arg
    370                 375                 380
Phe Lys Val Tyr Arg Gly Met Gly Ser Val Ala Ala Met Glu Lys Gly
385                 390                 395                 400
Ser Lys Asp Arg Tyr Phe Gln Glu Glu Asn Lys Lys Phe Val Pro Glu
                405                 410                 415
Gly Ile Glu Gly Arg Thr Pro Tyr Lys Gly Pro Val Glu Glu Thr Val
            420                 425                 430
Tyr Gln Leu Val Gly Gly Leu Arg Ser Gly Met Gly Tyr Cys Gly Ser
        435                 440                 445
Lys Asp Leu Arg Ala Leu Arg Glu Glu Ala Gln Phe Ile Arg Met Thr
    450                 455                 460
Gly Ala Gly Leu Arg Glu Ser His Pro His Asp Val Gln Ile Thr Val
465                 470                 475                 480
His Arg Asn Lys Ala Leu Pro Gly Leu Phe Gly Ser His Gln Lys Lys
                485                 490                 495
Thr Gly Phe Val Tyr Asp Glu Cys Cys Gln Ser Gly Phe Phe Ser Ser
            500                 505                 510
Asp

<210>  15
<211>  2013
<212>  DNA
<213>  Bacillus subtilis

<220>
<221>  CDS
<222>  (1)..(2013)

<400>  15
atg ttt aaa aat aat gtc ata ctt tta aat tca cct tat cat gca cat       48
Met Phe Lys Asn Asn Val Ile Leu Leu Asn Ser Pro Tyr His Ala His
1               5                   10                  15
gct cat aaa gag ggg ttt att cta aaa agg gga tgg acg gtt ttg gaa       96
Ala His Lys Glu Gly Phe Ile Leu Lys Arg Gly Trp Thr Val Leu Glu
            20                  25                  30
agc aag tac cta gat cta ctc gca caa aaa tac gat tgt gaa gaa aaa      144
Ser Lys Tyr Leu Asp Leu Leu Ala Gln Lys Tyr Asp Cys Glu Glu Lys
        35                  40                  45
gtg gta aca gaa atc atc aat ttg aaa gcg ata ttg aac ctg cca aaa      192
Val Val Thr Glu Ile Ile Asn Leu Lys Ala Ile Leu Asn Leu Pro Lys
    50                  55                  60
ggc acc gag cat ttt gtc agt gat ctg cac gga gag tat cag gca ttc      240
Gly Thr Glu His Phe Val Ser Asp Leu His Gly Glu Tyr Gln Ala Phe

65                  70                  75                  80
cag cac gtg ttg cgc aat ggt tca gga cga gtc aaa gag aag ata cgc      288
Gln His Val Leu Arg Asn Gly Ser Gly Arg Val Lys Glu Lys Ile Arg
                85                  90                  95
gac atc ttc agc ggt gtc att tac gat aga gaa att gat gaa tta gca      336
Asp Ile Phe Ser Gly Val Ile Tyr Asp Arg Glu Ile Asp Glu Leu Ala
            100                 105                 110
gca ttg gtc tat tat ccg gaa gac aaa ctg aaa tta atc aaa cat gac      384
Ala Leu Val Tyr Tyr Pro Glu Asp Lys Leu Lys Leu Ile Lys His Asp
        115                 120                 125
ttt gat gcg aaa gaa gcg tta aac gag tgg tat aaa gaa acg att cat      432
Phe Asp Ala Lys Glu Ala Leu Asn Glu Trp Tyr Lys Glu Thr Ile His
    130                 135                 140
cga atg att aag ctc gtt tca tat tgc tcc tct aag tat acc cgc tcc      480
Arg Met Ile Lys Leu Val Ser Tyr Cys Ser Ser Lys Tyr Thr Arg Ser
145                 150                 155                 160
aaa tta cgc aaa gca ctg cct gcc caa ttt gct tat att acg gag gag      528
Lys Leu Arg Lys Ala Leu Pro Ala Gln Phe Ala Tyr Ile Thr Glu Glu
                165                 170                 175
ctg tta tac aaa aca gaa caa gct ggc aac aag gag caa tat tac tcc      576
Leu Leu Tyr Lys Thr Glu Gln Ala Gly Asn Lys Glu Gln Tyr Tyr Ser
            180                 185                 190
gaa atc att gat cag atc att gaa ctt ggc caa gcc gat aag ctg atc      624
Glu Ile Ile Asp Gln Ile Ile Glu Leu Gly Gln Ala Asp Lys Leu Ile
        195                 200                 205
acc ggc ctt gct tac agc gtt cag cga ttg gtg gtc gac cat ctg cat      672
Thr Gly Leu Ala Tyr Ser Val Gln Arg Leu Val Val Asp His Leu His
    210                 215                 220
gtg gtc ggc gat att tat gac cgc ggc ccg cag ccg gat aga att atg      720
Val Val Gly Asp Ile Tyr Asp Arg Gly Pro Gln Pro Asp Arg Ile Met
225                 230                 235                 240
gaa gaa ctg atc aac tat cat tct gtc gat att cag tgg gga aat cac      768
Glu Glu Leu Ile Asn Tyr His Ser Val Asp Ile Gln Trp Gly Asn His
                245                 250                 255
gat gtc ctt tgg atc ggc gcc tat tcc ggt tcc aaa gtg tgc ctg gcc      816
Asp Val Leu Trp Ile Gly Ala Tyr Ser Gly Ser Lys Val Cys Leu Ala
            260                 265                 270
aat att atc cgc atc tgt gcc cgc tac gac aac ctg gat att att gag      864
Asn Ile Ile Arg Ile Cys Ala Arg Tyr Asp Asn Leu Asp Ile Ile Glu
        275                 280                 285
gac gtg tac ggc atc aac ctg aga ccg ctg ctg aac ctg gcc gaa aaa      912
Asp Val Tyr Gly Ile Asn Leu Arg Pro Leu Leu Asn Leu Ala Glu Lys
    290                 295                 300
tat tat gat gat aat cca gcg ttc cgt cca aaa gca gac gaa aac agg      960
Tyr Tyr Asp Asp Asn Pro Ala Phe Arg Pro Lys Ala Asp Glu Asn Arg
305                 310                 315                 320
cca gag gat gag att aag caa atc aca aaa atc cat caa gcg att gcc     1008
Pro Glu Asp Glu Ile Lys Gln Ile Thr Lys Ile His Gln Ala Ile Ala
                325                 330                 335
atg atc caa ttc aag ctt gag agc ccg att atc aag aga cgg ccg aac     1056
Met Ile Gln Phe Lys Leu Glu Ser Pro Ile Ile Lys Arg Arg Pro Asn
            340                 345                 350
ttt aat atg gaa gag cgg ctg tta tta gag aaa ata gac tat gac aaa     1104
Phe Asn Met Glu Glu Arg Leu Leu Leu Glu Lys Ile Asp Tyr Asp Lys
        355                 360                 365
aat gaa atc acg ctg aac gga aaa aca tat caa ctg gaa aac acc tgc     1152
Asn Glu Ile Thr Leu Asn Gly Lys Thr Tyr Gln Leu Glu Asn Thr Cys
    370                 375                 380
ttt gcg acg att aat ccg gag cag cca gat cag cta tta gaa gaa gaa     1200
Phe Ala Thr Ile Asn Pro Glu Gln Pro Asp Gln Leu Leu Glu Glu Glu
385                 390                 395                 400
gca gaa gtc ata gac aag ctg cta ttc tct gtc cag cat tcc gaa aag     1248
Ala Glu Val Ile Asp Lys Leu Leu Phe Ser Val Gln His Ser Glu Lys

405                    410                    415
ctg ggc cgc cat atg aat ttt atg atg aaa aaa ggc agc ctt tat tta      1296
Leu Gly Arg His Met Asn Phe Met Met Lys Lys Gly Ser Leu Tyr Leu
            420                    425                    430
aaa tat aac ggc aac ctg ttg att cac ggc tgt att cca gtt gat gaa      1344
Lys Tyr Asn Gly Asn Leu Leu Ile His Gly Cys Ile Pro Val Asp Glu
        435                    440                    445
aac ggc aat atg gaa acg atg atg att gag gat aaa ccg tat gcg ggc      1392
Asn Gly Asn Met Glu Thr Met Met Ile Glu Asp Lys Pro Tyr Ala Gly
    450                    455                    460
cgt gag ctg ctc gat gta ttt gaa cga ttc ttg cgg gaa gcc ttt gcc      1440
Arg Glu Leu Leu Asp Val Phe Glu Arg Phe Leu Arg Glu Ala Phe Ala
465                    470                    475                    480
cac ccg gaa gaa acc gat gac ctg gcg aca gat atg gct tgg tat tta      1488
His Pro Glu Glu Thr Asp Asp Leu Ala Thr Asp Met Ala Trp Tyr Leu
                485                    490                    495
tgg aca ggc gaa tac tcc tcc ctc ttc gga aaa cgc gcc atg acg aca      1536
Trp Thr Gly Glu Tyr Ser Ser Leu Phe Gly Lys Arg Ala Met Thr Thr
            500                    505                    510
ttt gag cgc tat ttc atc aaa gag aag gaa acg cat aaa gag aag aaa      1584
Phe Glu Arg Tyr Phe Ile Lys Glu Lys Glu Thr His Lys Glu Lys Lys
        515                    520                    525
aac ccg tat tat tat tta cga gaa gac gag gca acc tgc cga aac atc      1632
Asn Pro Tyr Tyr Tyr Leu Arg Glu Asp Glu Ala Thr Cys Arg Asn Ile
    530                    535                    540
ctg gca gaa ttc ggc ctc aat cca gat cac ggc cat atc atc aac ggc      1680
Leu Ala Glu Phe Gly Leu Asn Pro Asp His Gly His Ile Ile Asn Gly
545                    550                    555                    560
cat aca cct gta aaa gaa atc gaa gga gaa gac cca atc aaa gca aac      1728
His Thr Pro Val Lys Glu Ile Glu Gly Glu Asp Pro Ile Lys Ala Asn
                565                    570                    575
gga aaa atg atc gtc atc gac ggc ggc ttc tcc aaa gcc tac caa tcc      1776
Gly Lys Met Ile Val Ile Asp Gly Gly Phe Ser Lys Ala Tyr Gln Ser
            580                    585                    590
aca aca ggc atc gcc ggc tac acg ctg cta tac aac tcc tac ggc atg      1824
Thr Thr Gly Ile Ala Gly Tyr Thr Leu Leu Tyr Asn Ser Tyr Gly Met
        595                    600                    605
cag ctc gtc gcc cat aaa cac ttc aat tcc aag gca gaa gtc cta agc      1872
Gln Leu Val Ala His Lys His Phe Asn Ser Lys Ala Glu Val Leu Ser
    610                    615                    620
acc gga acc gac gtc tta acg gtc aaa cga tta gtg gac aaa gag ctt      1920
Thr Gly Thr Asp Val Leu Thr Val Lys Arg Leu Val Asp Lys Glu Leu
625                    630                    635                    640
gag cgg aag aaa gtg aag gaa acg aat gtg ggt gag gaa ttg ttg cag      1968
Glu Arg Lys Lys Val Lys Glu Thr Asn Val Gly Glu Glu Leu Leu Gln
                645                    650                    655
gaa gtt gcg att tta gag agt ttg cgg gag tat cgg tat atg aag          2013
Glu Val Ala Ile Leu Glu Ser Leu Arg Glu Tyr Arg Tyr Met Lys
            660                    665                    670

<210>  16
<211>  671
<212>  PRT
<213>  Bacillus subtilis

<400>  16
Met Phe Lys Asn Asn Val Ile Leu Leu Asn Ser Pro Tyr His Ala His
1               5                   10                  15
Ala His Lys Glu Gly Phe Ile Leu Lys Arg Gly Trp Thr Val Leu Glu
            20                  25                  30
Ser Lys Tyr Leu Asp Leu Leu Ala Gln Lys Tyr Asp Cys Glu Glu Lys
        35                  40                  45
Val Val Thr Glu Ile Ile Asn Leu Lys Ala Ile Leu Asn Leu Pro Lys

50                  55                  60
Gly Thr Glu His Phe Val Ser Asp Leu His Gly Glu Tyr Gln Ala Phe
65                  70                  75                  80
Gln His Val Leu Arg Asn Gly Ser Gly Arg Val Lys Glu Lys Ile Arg
                85                  90                  95
Asp Ile Phe Ser Gly Val Ile Tyr Asp Arg Glu Ile Asp Glu Leu Ala
            100                 105                 110
Ala Leu Val Tyr Tyr Pro Glu Asp Lys Leu Lys Leu Ile Lys His Asp
        115                 120                 125
Phe Asp Ala Lys Glu Ala Leu Asn Glu Trp Tyr Lys Glu Thr Ile His
    130                 135                 140
Arg Met Ile Lys Leu Val Ser Tyr Cys Ser Ser Lys Tyr Thr Arg Ser
145                 150                 155                 160
Lys Leu Arg Lys Ala Leu Pro Ala Gln Phe Ala Tyr Ile Thr Glu Glu
                165                 170                 175
Leu Leu Tyr Lys Thr Glu Gln Ala Gly Asn Lys Glu Gln Tyr Tyr Ser
            180                 185                 190
Glu Ile Ile Asp Gln Ile Ile Glu Leu Gly Gln Ala Asp Lys Leu Ile
        195                 200                 205
Thr Gly Leu Ala Tyr Ser Val Gln Arg Leu Val Val Asp His Leu His
    210                 215                 220
Val Val Gly Asp Ile Tyr Asp Arg Gly Pro Gln Pro Asp Arg Ile Met
225                 230                 235                 240
Glu Glu Leu Ile Asn Tyr His Ser Val Asp Ile Gln Trp Gly Asn His
                245                 250                 255
Asp Val Leu Trp Ile Gly Ala Tyr Ser Gly Ser Lys Val Cys Leu Ala
            260                 265                 270
Asn Ile Ile Arg Ile Cys Ala Arg Tyr Asp Asn Leu Asp Ile Ile Glu
        275                 280                 285
Asp Val Tyr Gly Ile Asn Leu Arg Pro Leu Leu Asn Leu Ala Glu Lys
    290                 295                 300
Tyr Tyr Asp Asp Asn Pro Ala Phe Arg Pro Lys Ala Asp Glu Asn Arg
305                 310                 315                 320
Pro Glu Asp Glu Ile Lys Gln Ile Thr Lys Ile His Gln Ala Ile Ala
                325                 330                 335
Met Ile Gln Phe Lys Leu Glu Ser Pro Ile Ile Lys Arg Arg Pro Asn
            340                 345                 350
Phe Asn Met Glu Glu Arg Leu Leu Leu Glu Lys Ile Asp Tyr Asp Lys
        355                 360                 365
Asn Glu Ile Thr Leu Asn Gly Lys Thr Tyr Gln Leu Glu Asn Thr Cys
    370                 375                 380
Phe Ala Thr Ile Asn Pro Glu Gln Pro Asp Gln Leu Leu Glu Glu Glu
385                 390                 395                 400
Ala Glu Val Ile Asp Lys Leu Leu Phe Ser Val Gln His Ser Glu Lys
                405                 410                 415
Leu Gly Arg His Met Asn Phe Met Met Lys Lys Gly Ser Leu Tyr Leu
            420                 425                 430
Lys Tyr Asn Gly Asn Leu Leu Ile His Gly Cys Ile Pro Val Asp Glu
        435                 440                 445
Asn Gly Asn Met Glu Thr Met Met Ile Glu Asp Lys Pro Tyr Ala Gly
    450                 455                 460
Arg Glu Leu Leu Asp Val Phe Glu Arg Phe Leu Arg Glu Ala Phe Ala
465                 470                 475                 480
His Pro Glu Glu Thr Asp Asp Leu Ala Thr Asp Met Ala Trp Tyr Leu
                485                 490                 495
Trp Thr Gly Glu Tyr Ser Ser Leu Phe Gly Lys Arg Ala Met Thr Thr
            500                 505                 510
Phe Glu Arg Tyr Phe Ile Lys Glu Lys Glu Thr His Lys Glu Lys Lys
        515                 520                 525
Asn Pro Tyr Tyr Tyr Leu Arg Glu Asp Glu Ala Thr Cys Arg Asn Ile
    530                 535                 540
Leu Ala Glu Phe Gly Leu Asn Pro Asp His Gly His Ile Ile Asn Gly
545                 550                 555                 560

His Thr Pro Val Lys Glu Ile Glu Gly Glu Asp Pro Ile Lys Ala Asn
                565                 570                 575
Gly Lys Met Ile Val Ile Asp Gly Gly Phe Ser Lys Ala Tyr Gln Ser
            580                 585                 590
Thr Thr Gly Ile Ala Gly Tyr Thr Leu Leu Tyr Asn Ser Tyr Gly Met
        595                 600                 605
Gln Leu Val Ala His Lys His Phe Asn Ser Lys Ala Glu Val Leu Ser
    610                 615                 620
Thr Gly Thr Asp Val Leu Thr Val Lys Arg Leu Val Asp Lys Glu Leu
625                 630                 635                 640
Glu Arg Lys Lys Val Lys Glu Thr Asn Val Gly Glu Glu Leu Leu Gln
                645                 650                 655
Glu Val Ala Ile Leu Glu Ser Leu Arg Glu Tyr Arg Tyr Met Lys
            660                 665                 670

<210>  17
<211>  17
<212>  DNA
<213>  Artificial

<220>
<223>  primer

<400>  17
gaagttgatgatcaaaa                                                    17

<210>  18
<211>  19
<212>  DNA
<213>  Artificial

<220>
<223>  primer

<400>  18
acatattgtt gacgataat                                                 19

<210>  19
<211>  28
<212>  DNA
<213>  Artificial

<220>
<223>  primer

<400>  19
ttcccttagg gttattttcg tttcaaaa                                       28

<210>  20
<211>  50
<212>  DNA
<213>  Artificial

<220>
<223>  primer

<400>  20
cgtttgttga actaatgggt gcttttatga gcatgtgcat gataaggtga               50

<210>  21
<211>  50
<212>  DNA
<213>  Artificial

<220>
<223>  primer

<400>  21
acagctccag atccatatcc ttctttttta gagagtttgc gggagtatcg          50

<210>  22
<211>  27
<212>  DNA
<213>  Artificial

<220>
<223>  primer

<400>  22
taaaggtttt tcgggataag attgaaa                                  27

<210>  23
<211>  50
<212>  DNA
<213>  Artificial

<220>
<223>  primer

<400>  23
tcaccttatc atgcacatgc tcataaaagc acccattagt tcaacaaacg          50

<210>  24
<211>  50
<212>  DNA
<213>  Artificial

<220>
<223>  primer

<400>  24
cgatactccc gcaaactctc taaaaaagaa ggatatggat ctggagctgt          50

<210>  25
<211>  28
<212>  DNA
<213>  Artificial

<220>
<223>  primer

<400>  25
atggacggaa tcgaaatctt aaaacgga                                 28

<210>  26
<211>  50
<212>  DNA
<213>  Artificial

<220>
<223>  primer

<400>  26
cgtttgttga actaatgggt gctttagaat tcctaattca ttcgcttctc          50

<210>  27

<211>  50
<212>  DNA
<213>  Artificial

<220>
<223>  primer

<400>  27
acagctccag atccatatcc ttctttgacc tgatttcaga agttaaacag          50

<210>  28
<211>  28
<212>  DNA
<213>  Artificial

<220>
<223>  primer

<400>  28
ggatgacgtt atcgataatg acttcctt                                  28

<210>  29
<211>  50
<212>  DNA
<213>  Artificial

<220>
<223>  primer

<400>  29
gagaagcgaa tgaattagga attctaaagc acccattagt tcaacaaacg          50

<210>  30
<211>  50
<212>  DNA
<213>  Artificial

<220>
<223>  primer

<400>  30
ctgtttaact tctgaaatca ggtcaaagaa ggatatggat ctggagctgt          50

<210>  31
<211>  26
<212>  DNA
<213>  Artificial

<220>
<223>  primer

<400>  31
taaagcgtga tagacataca gtgctg                                    26

<210>  32
<211>  50
<212>  DNA
<213>  Artificial

<220>
<223>  primer

<400>  32

cattgcaaga cttttttcac caagcagaat tcctaattca ttcgcttctc          50

<210>  33
<211>  50
<212>  DNA
<213>  Artificial

<220>
<223>  primer

<400>  33
gagagagttc aaaattgatc ctttttgacc tgatttcaga agttaaacag          50

<210>  34
<211>  26
<212>  DNA
<213>  Artificial

<220>
<223>  primer

<400>  34
ttgcatatac atgtcaggag cattca                                    26

<210>  35
<211>  50
<212>  DNA
<213>  Artificial

<220>
<223>  primer

<400>  35
gagaagcgaa tgaattagga attctgcttg gtgaaaaaag tcttgcaatg          50

<210>  36
<211>  50
<212>  DNA
<213>  Artificial

<220>
<223>  primer

<400>  36
ctgtttaact tctgaaatca ggtcaaaaag gatcaatttt gaactctctc          50

## Description

### Technical Field

The present invention relates to a method for producing purine-derived substances such as purine nucleotides typically including 5'-inosinic acid and 5'-guanylic acid, purine nucleosides such as inosine and guanosine, which are important substances as starting materials for synthesis of the purine nucleotides, and so forth, and a bacterium belonging to the genus *Bacillus* used for the method. Purine-derived substances are useful as seasonings, drugs, raw materials thereof, and so forth.

### Background Art

Methods for producing inosine and guanosine by fermentation using adenine auxotrophic strains of *Bacillus* bacteria and derivatives thereof which are further imparted with resistance to various drugs such as purine analogues (Patent documents 1 to 8), and such microorganisms of the genus *Brevibacterium* (Patent documents 9 and 10, Non-patent document 1), and so forth have been known.

Such mutant strains are typically obtained by treating a microorganism with ultraviolet irradiation or nitrosoguanidine (N-methyl-N'-nitro-N-nitrosoguanidine), and selecting a target mutant using a suitable selection medium.

Furthermore, strains which produce purine-derived substances have also been bred using genetic engineering techniques in *Bacillus* bacteria (Patent documents 11 to *20), Brevibacterium* bacteria(Patent document 21), and *Escherichia* bacteria (Patent document 22). Specifically, for example, a method for efficiently producing a nucleic acid-derived compound such as hypoxanthine, uracil, guanine and adenine with a *Bacillus* bacterium in which a gene (*purR*) encoding the purine operon repressor is disrupted is disclosed (Patent document 23).

In *Bacillus subtilis,* the purine operon repressor as described above is known to regulate, besides the genes of purine operon, the *purA* gene which is involved in AMP biosynthesis (Non-patent document 2), the *glyA* gene which is involved in formyltetrahydrofolic acid biosynthesis (Non-patent document 3), the *pbuG* gene which encodes the transporter of hypoxanthine/guanine (Non-patent document 4), and so forth.

Furthermore, a microorganism which efficiently produces inosine derived by impartation of adenine auxotrophy by disruption of the succinyl-AMP synthase gene (*purA*) in addition to disruption of the *purR* gene, and suppression of decomposition of inosine into hypoxanthine by disruption of the purine nucleoside phosphorylase gene *(deoD),* and a method for producing inosine using the microorganism have been disclosed (see Patent document 8).

Transaldolase is one of the enzymes of the pentose phosphate pathway, which catalyzes the reversible reaction generating D-erythrose-4-phosphate and D-fructose-6-phosphate from sedoheptulose-7-phosphate and D-glyceraldehyde-3-phosphate. There is only little knowledge about the relationship between this enzyme and the biosynthetic pathway of purine-derived substances, and it has not been attempted to breed a purine-derived substance-producing bacterium by reducing the activity of this enzyme.
Patent document 1: Japanese Patent Publication (KOKOKU) No. 38-23099
Patent document 2: Japanese Patent Publication No. 54-17033
Patent document 3: Japanese Patent Publication No. 55-2956
Patent document 4: Japanese Patent Publication No. 55-45199
Patent document 5: Japanese Patent Publication No. 57-14160
Patent document 6: Japanese Patent Publication No. 57-41915
Patent document 7: Japanese Patent Laid-open (KOKAI) No. 59-42895
Patent document 8: Japanese Patent Laid-open No. 2004-242610
Patent document 9: Japanese Patent Publication No. 51-5075
Patent document 10: Japanese Patent Publication No. 58-17592
Patent document 11: Japanese Patent Laid-open No. 58-158197
Patent document 12: Japanese Patent Laid-open No. 58-175493
Patent document 13: Japanese Patent Laid-open No. 59-28470
Patent document 14: Japanese Patent Laid-open No. 60-156388
Patent document 15: Japanese Patent Laid-open No. 1-27477
Patent document 16: Japanese Patent Laid-open No. 1-174385
Patent document 17: Japanese Patent Laid-open No. 3-58787
Patent document 18: Japanese Patent Laid-open No. 3-164185
Patent document 19: Japanese Patent Laid-open No. 5-84067
Patent document 20: Japanese Patent Laid-open No. 5-192164
Patent document 21: Japanese Patent Laid-open No. 63-248394
Patent document 22: International Patent Publication WO99/03988
Patent document 23: U.S. Patent No. 6,284,495
Non-patent document 1: Agric. Biol. Chem., 1978, 42, 399-405
Non-patent document 2: Proc. Natl. Acad. Sci. USA, 1995, 92, 7455-7459
Non-patent document 3: J. Bacteriol., 2001, 183, 6175-6183 Non-patent document 4: J. Bacteriol., 2003, 185, 5200-5209

### Disclosure of the Invention

### Object to be Achieved by the Invention

An object of the present invention is to create a *Bacillus* bacterium suitable for production of purine-derived substances such as purine nucleosides and/or purine nucleotides by fermentation, and to provide a method for producing a purine-derived substance using such a bacterium.

### Means for Achieving the Object

The inventors of the present invention conducted various researches in order to achieve the aforementioned object. As a result, they found that if the enzymatic activity of transaldolase of the pentose phosphate pathway was decreased in a *Bacillus* bacterium, an ability of the bacterium to produce a purine nucleoside or a purine nucleotide was improved, and accomplished the present invention.

The present invention thus provides the followings.
(1) A bacterium belonging to the genus *Bacillus* having an ability to produce purine-derived substance wherein the bacterim has been modified so that enzymatic activity of transaldolase is decreased.
(2) The bacterium belonging to the genus *Bacillus,* wherein the purine-derived substance is purine nucleoside selected from the group consisting of inosine, xanthosine, guanosine and adenosine.
(3) The bacterium belonging to the genus *Bacillus* as described above, wherein the purine-derived substance is purine nucleotide selected from the group consisting of inosinic acid, xanthylic acid, guanylic acid and adenylic acid.
(4) The bacterium belonging to the genus *Bacillus* as described above, wherein the transaldolase activity is decreased by disruption of a gene encoding transaldolase, or reducing expression amount of the gene encoding transaldolase.
(5) The bacterium belonging to the genus *Bacillus* as described above, wherein the gene encoding transaldolase is a gene encoding a protein of the following (A) or (B):
   (A) a protein comprising the amino acid sequence of SEQ ID NO: 1,
   (B) a protein comprising an amino acid sequence of SEQ ID NO: 1 which includes substitutions, deletions, insertions, additions or inversions of one or several amino acid residues and has transaldolase activity.
(6) The bacterium belonging to the genus *Bacillus* as described above, which is further modified so that phosphoribosyl pyrophosphate synthetase activity should increase.
(7) The bacterium belonging to the genus *Bacillus* as described above, which has been further modified so that expression amount of the purine operon is increased.
(8) The *Bacillus* as described above, wherein expression amount of the purine operon is increased by disruption of the *purR* gene, which is a gene encoding a repressor of the purine operon, or deletion of a part of attenuator region of the purine operon.
(9) The bacterium belonging to the genus *Bacillus* as described above, which has been further modified so that purine nucleoside phosphorylase activity is decreased.
(10) The bacterium belonging to the genus *Bacillus* as described above, which has been further modified so that fructose bisphosphatase activity is decreased.
(11) The bacterium belonging to the genus *Bacillus* as described above, which has been further modified so that IMP dehydrogenase activity is decreased.
(12) The bacterium belonging to the genus *Bacillus* as described above, which is *Bacillus subtilis.*
(13) A method for producing a purine-derived substance, which comprises culturing the bacterium belonging to the genus *Bacillus* as described above in a medium to cause accumulation of a purine-derived substance in cells of the bacterium or the medium, and collecting the purine-derived substance from the cells or medium.
(14) The method as described above, wherein the purine-derived substance is a purine nucleoside or a purine nucleotide.
(15) The method as described above, wherein the purine-derived substance is a purine nucleoside selected from the group consisting of inosine, xanthosine, guanosine and adenosine.
(16) The method as described above, wherein the purine-derived substance is a purine nucleotide selected from the group consisting of inosinic acid, xanthylic acid, guanylic acid and adenylic acid.
(17) A method for producing a purine nucleotide, which comprises producing a purine nucleoside by the method as described above, reacting the purine nucleoside with a phosphate donor selected from the group consisting of polyphosphoric acid, phenyl phosphate and carbamyl phosphate, and a microorganism having an ability to produce a nucleoside-5'-phosphoric acid ester or acid phosphatase to produce a purine nucleotide, and collecting the purine nucleotide.

### Best Mode for Carrying out the Invention

### <1> Bacillus bacterium of the present invention

### (I) Impartating the ability to produce a purine-derived substance

The *Bacillus* bacterium of the present invention is a *Bacillus* bacterium having an ability to produce a purine-derived substance and has been modified so that enzymatic activity of transaldolase is decreased.

The term "purine-derived substance" means a substance having a purine skelton, and examples thereof include purine nucleosides, purine nucleotides, and so forth. The purine nucleosides include inosine, xanthosine, guanosine, adenosine, and so forth, and the purine nucleotides include 5'-phosphoric acid esters of purine nucleosides, for example, inosinic acid (inosine-5'-phosphate, henceforth also referred to as "IMP"), xanthylic acid (xanthosine-5'-phosphate, henceforth also referred to as "XMP"), guanylic acid (guanosine-5'-monophosphate, henceforth also referred to as "GMP"), adenylic acid (adenosine-5'-monophosphate, henceforth also referred to as "AMP"), and so forth.

The phrase "ability to produce a purine-derived substance" means an ability of the *Bacillus* bacterium of the present invention to produce, secrete or cause accumulation of a purine-derived substance in the bacterial cells or a medium when the bacterium is cultured in the medium to such an extent that the purine-derived substance can be collected from the cells or medium. The *Bacillus* bacterium of the present invention may have an ability to produce two or more kinds of the aforementioned purine-derived substances.

The *Bacillus* bacterium having the ability to produce a purine-derived substance may be a bacterium inherently having the ability to produce a purine-derived substance, or a bacterium obtainable by modifying such *Bacillus* bacteria as mentioned below using a mutagenesis method or recombinant DNA technique so that they have the ability to produce a purine-derived substance. Moreover, it may be a *Bacillus* bacterium to which the ability to produce a purine-derived substance is imparted or of which the ability to produce a purine-derived substance is enhanced by modifying the bacterium so that enzymatic activity of transaldolase is decreased, in such a manner as described later.

In the present invention, the phrase "enzymatic activity is decreased" encompasses a state that enzymatic activity of transaldolase mentioned above, or an enzyme which decomposes a purine-derived substance mentioned later such as inosine monophosphate (IMP) dehydrogenase is lower than that in an unmodified strain, for example, a wild-type strain of the *Bacillus* bacterium, and a state that the activity has substantially disappeared. The same shall apply to the activity of the purine operon repressor described later.

Examples of the *Bacillus* bacterium used for obtaining the *Bacillus* bacterium of the present invention include *Bacillus subtilis, Bacillus amyloliquefaciens, Bacillus pumilus,* and so forth.

Examples of *Bacillus subtilis* include *Bacillus subtilis* 168 Marburg (ATCC 6051), *Bacillus subtilis* PY79 (Plasmid, 1984, 12, 1-9) and so forth, and examples of *Bacillus amyloliquefaciens* include *Bacillus amyloliquefaciens* T (ATCC 23842), *Bacillus amyloliquefaciens N* (ATCC 23845), and so forth. Examples of *Bacillus pumilus* include *Bacillus pumilus* Gottheil No. 3218 (ATCC No. 21005, U.S. Patent No. 3,616,206), and so forth. These strains can be obtained from American Type Culture Collection (Address: P.O. Box 1549, Manassas, VA 20108, United States of America).

A *Bacillus* bacterium having the ability to produce a purine-derived substance can be obtained by imparting, for example, purine nucleoside auxotrophy or resistance to a drug such as purine analogue to such *Bacillus* bacteria as mentioned above (Japanese Patent Publication Nos. 38-23099, 54-17033, 55-45199, 57-14160, 57-41915 and 59-42895). A *Bacillus* bacterium having auxotrophy or drug resistance can be obtained by treating the bacterium with mutatgen which is used for usual mutagenesis treatment such as N-methyl-N'-nitro-N-nitrosoguanidine (NTG) or EMS (ethyl methanesulfonate).

Examples of *Bacillus* bacteria which produce a purine nucleoside include the followings.

As a specific example of inosine-producing strain belonging to the genus *Bacillus,* the *Bacillus subtilis* KMBS16 strain can be used. This strain is derived from the known *Bacillus subtilis* trpC2 strain (168 Marburg), wherein a *purR* gene encoding the purine operon repressor (purR::spc), the *purA* gene encoding succinyl-AMP synthase (purA::erm), and the deoD gene encoding purine nucleoside phosphorylase (deoD::kan) are disrupted (Japanese Patent Laid-open No. 2004-242610, US2004166575A1). *Bacillus subtilis* AJ3772 strain (FERM P-2555, Japanese Patent Laid-open No. 62-014794) and so forth may also be used.

Examples of *Bacillus* bacteria has an ability to prouduce guanosine include a *Bacillus* bacterium which has increased IMP dehydrogenase activity (Japanese Patent Laid-open No. 3-58787), a *Bacillus* bacterium which is obtained by introducing a vector comprising a gene conferring resistance to purine analogue or decoyinine into an adenine auxotrophic mutant (Japanese Patent Publication No. 4-28357), and so forth.

Examples of *Bacillus* bacteria which produce a purine nucleotide include the followings.

As an inosinic acid-producing *Bacillus* bacteria, inosine-producing strains of *Bacillus subtilis* which have attenuated phosphatase activity have been reported (Uchida, K. et al., Agr. Biol. Chem., 1961, 25, 804-805; Fujimoto, M., Uchida, K., Agr. Biol. Chem., 1965, 29, 249-259). Examples of guanylic acid-producing *Bacillus* bacteria include mutants of *Bacillus* bacteria which have adenine auxotrophy, resistance to decoyinine or methionine sulfoxide and an ability to produce 5'-guanylic acid (guanosine-5'-monophosphate, henceforth referred to as "GMP") (Japanese Patent Publication No. 56-12438).

Furthermore, a xanthylic acid-producing bacterium can be constructed by the method used for breeding of coryneform bacteria of which typical example is *Corynebacterium ammoniagenes.* For example, by obtaining a PRPP amidotransferase-enhanced strain (Japanese Patent Laid-open No. 8-168383), an aliphatic amino acid-resistant strain (Japanese Patent Laid-open No. 4-262790), or a dehydroproline-resistant strain (South Korean Patent Unexamined Publication No. 2003-56490), a xanthylic acid-producing bacterium can be constructed.

Moreover, example of a method for breeding *Bacillus* bacteria which have the ability to produce a purine-derived substance also includes enhancing the activity of enzymes which involved in purine biosynthesis which are common to the biosynthesis of purine nucleosides and purine nucleotides, i.e., purine biosynthesis enzymes, in bacterial cells. The activity of the enzyme in the cells is preferably increased to a level greater than that of an unmodified strain of *Bacillus* bacterium, for example, a wild-type *Bacillus* bacterium. The phrase "activity is increased" encompasses, for example, when number of enzyme molecules per cell is increased, and when specific activity per enzyme molecule is increased, and so forth. For example, the activity can be increased by increasing expression amount of the gene of the enzyme.

Examples of an enzyme involved in the purine biosynthesis include, for example, phosphoribosyl pyrophosphate amidotransferase, phosphoribosyl pyrophosphate synthetase (PRPP synthetase [EC: 2.7.6.1]), and so forth.

Some of the catabolites produced by metabolism of sugar sources such as glucose that flow into the pentose phosphate pathway are converted into ribose-5-phosphate via ribulose-5-phosphate. From the biosynthesized ribose-5-phosphate, phosphoribosyl pyrophosphate (PRPP) is produced, which is an indispensable precursor for purine nucleoside, histidine and tryptophan biosyntheses. Specifically, ribose-5-phosphate is converted into PRPP by phosphoribosyl pyrophosphate synthetase. Therefore, an ability to produce purine-derived substance can be imparted to a *Bacillus* bacterium by modifying so that the activity of phosphoribosyl pyrophosphate synthetase thereof is increased.

The phrase "activity of phosphoribosyl pyrophosphate synthetase is increased" means that the activity of phosphoribosyl pyrophosphate synthetase increases as compared to that of an unmodified strain such as a wild strain or a parent strain. The activity of the phosphoribosyl pyrophosphate synthetase can be measured by, for example, the method of Switzer et al. (Methods Enzymol., 1978, 51, 3-11) or Roth et al. (Methods Enzymol., 1978, 51, 12-17). A *Bacillus* bacterium in which the activity of phosphoribosyl pyrophosphate synthetase is increased can be produced by, for example, increasing expression of a gene encoding the phosphoribosyl pyrophosphate synthetase in a *Bacillus* bacterium according to a method of using a plasmid containing the gene or integrating the gene into a chromosome, which can be performed in the same manner as that of the method described in Japanese Patent Laid-open No. 2004-242610. Although the *prs* gene of SEQ ID NO: 3 derived from a *Bacillus* bacterium (Genbank Accession No. X16518) can be mentioned as the gene encoding phosphoribosyl pyrophosphate synthetase usable for the present invention, any of genes derived from other bacteria, animals or plants encoding a protein having the phosphoribosyl pyrophosphate synthetase activity may be used.

Furthermore, when PRPP which is an indispensable precursor for purine nucleoside, histidine and tryptophan biosyntheses is once produced, some of it is converted into purine nucleotides and purine nucleosides by the enzymes involved in the purine biosynthesis. Examples of genes encoding such enzymes include the genes of the purine operon from *Bacillus subtilis,* specifically, genes of the purEKB-purC(orf) QLF-purMNH(J)-purD operon (Ebbole D.J. and Zalkin H., J. Biol. Chem., 1987, 262, 17, 8274-87) (at present, also called purEKBCSQLFMNHD, Bacillus subtilis and Its Closest Relatives, Editor in Chief: A.L. Sonenshein, ASM Press, Washington D.C., 2002, Genbank Accession No. NC_000964), and the genes of the pur regulon from *Escherichia coli (*Escherichia and Salmonella, Second Edition, Editor in Chief: F.C. Neidhardt, ASM Press, Washington D.C., 1996).

Accordingly, enhancing expression of these genes imparts or enhances the ability to produce a purine-derived substance can be imparted or enhanced. In addition, genes of the purine operon which can be used for the present invention are not limited to these, and genes derived from other microorganisms, animals and plants may also be used.

Examples of the method for increasing expression amount of the purine operon include increasing expression of genes of the purine operon genes in a *Bacillus* bacterium by a method of using a plasmid containing the genes or integrating the genes into a chromosome or the like.

The second method for increasing expression amount of the purine operon includes, replacing a promoter of the purine operon with a stronger promoter, and replacing the - 35 or -10 region of the native promoter with a consensus sequence.

For example, in *Bacillus subtilis (B. subtilis* 168 Marburg strain, ATCC 6051), the -35 sequence of the purine operon is a consensus sequence (TTGACA), but the -10 sequence is TAAGAT, which differs from the consensus sequence TATAAT (Ebbole, D.J. and H. Zalikn, J. Biol. Chem., 1987, 262, 8274-8287). Therefore, by changing the -10 sequence (TAAGAT) to the similar consensus sequence TATAAT, TATGAT or TAAAAT, the transcriptional activity of the purine operon can be increased. A promoter sequence can be replaced by the same method as that of the gene substitution, which is described below.

The third method for increasing amount of expression of the purine operon includes reducing expression amount of the purine operon repressor (U.S. Patent No. 6,284,495). The phrase "expression of a purine operon repressor" includes both transcription of a purine operon gene and translation of a transcription product. Furthermore, "expression amount is decreased" include when the expression amount is lower than that in an unmodified strain such as a wild-type *Bacillus* bacterium, and when the expression has substantially eliminated.

Expression amount of the purine operon repressor (purine repressor) can be decreased by, for example, treating a *Bacillus* bacterium with ultraviolet ray irradiation or mutagen used in a usual mutagenesis treatment such as NTG or EMS and selecting a mutant showing decreased expression amount of the purine repressor can be employed.

Furthermore, a *Bacillus* bacterium exhibiting a decreased expression amount of the purine repressor can also be obtained by, for example, besides a mutagenesis treatment, replacing a gene encoding the purine repressor on a chromosome (*purR,* GenBank Accession NC_000964, coding region corresponds to the nucleotide numbers 54439 to 55293, SEQ ID NO: 5) with a corresponding gene that does not normally function (hereafter, also referred to as "disrupted-type gene") by homologous recombination utilizing a gene recombination technique (Experiments in Molecular Genetics, Cold Spring Harbor Laboratory Press (1972); Matsuyama, S. and Mizushima, S., J. Bacteriol., 1985, 162, 1196-1202).

For example, a disrupted-type gene for a normal gene can be replaced with a disrupted-type gene on the host chromosome in the mannaer as described below. Herein after, the disruption of the *purR* gene is explained. Other genes such as *purA, deoD, guaB, fbp* and *ywjH* genes can be similarly disrupted.

When a plasmid or the like that has a sequence homologous with a sequence on the chromosome and which is not capable of replicating in cells of *Bacillus* bacteria is introduced into the cell, resulting in recombination at the site of the sequence having homology at a certain frequency. The entire plasmid is then recombined into the chromosome. Thereafter, if recombination is further caused at the site of the sequence having homology on the chromosome, the plasmid is removed from the chromosome. At this time, depending on the site where the recombination occurs, the disrupted-type gene may be kept on the chromosome, and the original normal gene may be removed from the chromosome with the plasmid. By selecting such a strain, it is possible to obtain a strain in which the normal *purR* gene on the chromosome has been replaced with the disrupted-type *purR* gene.

Such gene disruption techniques based on homologous recombination has already been established and include a method of utilizing a linear DNA, a method of utilizing a temperature sensitive plasmid and so forth. Furthermore, the *purR* gene can also be disrupted by using a plasmid which contains the *purR* gene into which a marker gene such as a drug resistance gene has been inserted and which is not able to replicate in the target bacterial cell. That is, in a transformant that has been transformed with such a plasmid as described above, the marker gene is incorporated into the chromosomal DNA and imparts drug resitance. Since the marker gene is incorporated into the chromosome at a hige rate by homologous recombination of the *purR* gene sequences that sandwiches the marker gene on the plasmid with the *purR* gene on the chromosome, a *purR* gene-disrupted strain can be efficiently selected.

The disrupted-type *purR* gene used for the gene disruption can be obtained by, specifically, deleting a certain region of the *purR* gene by digestion with a restriction enzyme and re-ligation, inserting another DNA fragment (marker gene etc.) into the *purR* gene, or causing substitution, deletion, insertion, addition or inversion of one or more nucleotides in the nucleotide sequence of the coding region, promoter region or the like of the *purR* gene by a site-specific mutagenesis (Kramer, W. and Frits, H.J., Methods in Enzymology, 1987, 154, 350-367) or by recombinant PCR (PCR Technology, Stockton Press (1989)) or by treantment with a chemical agent such as sodium hyposulfite or hydroxylamine (Shortle, D. and Nathans, D., Proc. Natl. Acad. Sci. U.S.A., 1978, 75, 2170-2174), followed by selection of a strain in which the activity of the repressor encoded by the *purR* gene is decreased or transcription of the gene is decreased. Among these methods, deleting a certain region of the *purR* gene by digestion with a restriction enzyme and re-ligation or inserting another DNA fragment into the *purR* gene is preferable in view of reliability and stability. The certain region of the *purR* gene to be deleted may be a 5' end sequence, internal sequence or 3' end sequence of the *purR* gene. However, if the region accounts for 90% or more, more preferably 95% or more, particularly 97% or more, of the full length of the *purR* gene, certainty of reduction of the repressor activity is increased. Furthermore, when a frame shift mutation is caused by deletion or insertion of nucleotides in the coding region of the *purR* gene, it is preferable to cause deletion or insertion of nucleotides at multiple sites and cause deletion or insertion of nucleotides on the 3' end side, in view of surely reducing the repressor activity.

Reduction of the purine repressor activity can also be attained by, besides the aforementioned gene disruption, introduction of a mutation that reduces the intracellular purine repressor activity into the *purR* gene on the chromosome based on a conventional mutagenesis method. For example, it can be attained by introducing an amino acid substitution (missense mutation), a stop codon (nonsense mutation), or a frame shift mutation that adds or deletes one or two nucleotides, or by deleting the gene partially or entirely. Furthermore, the activity of the repressor can also be decreased by inserting a transposon into the *purR* gene on the chromosome.

Reduction of the activity of the purine repressor can also be attained by replacing an expression control sequence of the *purR* gene such as promoter on the chromosomal DNA with a weaker one. The strength of a promoter is defined by the frequency of initiation of RNA synthesis. Examples of method for evaluating the strength of promoters and strong promoters are described in the paper of Goldstein et al. (Prokaryotic promoters in biotechnology, Biotechnol. Annu. Rev., 1995, 1, 105-128), and so forth. Furthermore, it is also possible to introduce nucleotide substitution for several nucleotides in a promoter region of a target gene and thereby modify the promoter to be weakened as disclosed in International Patent Publication WO00/18935. Furthermore, it is known that substitution of several nucleotides in the spacer between the ribosome binding site (RBS) and the start codon, in particular, a sequence immediately upstream from the start codon, greatly affects translation efficiency of mRNA. This modification of RBS may be combined with decreasing transcription of a target gene.

Furthermore, a recombinant DNA into which a mutation that unstabilizes messenger RNA transcribed from the *purR* gene is introduced may be prepared and transformed into a host *Bacillus* bacterium.

Activities of enzymes encoded by the *purA, deoD, guaB,* fbp and *ywjH* genes described later can also be decreased in the same manner as described above.

The *purR* gene can be obtained from a chromosomal DNA of a microorganism having the purine operon by PCR using oligonucleotides prepared based on the known nucleotide. sequence of the *purR* gene as primers. The *purR* gene can also be obtained from a chromosomal DNA library of a microorganism having the purine operon by hybridization using an oligonucleotide prepared on the basis of the known nucleotide sequence of the *purR* gene as a probe. The nucleotide sequence of the *purR* gene of the *Bacillus subtilis* 168 Marburg strain has been reported (GenBank accession No. D26185 (the coding region corresponds to the nucleotide numbers 118041 to 118898), or NC_000964 (the coding region corresponds to the nucleotide numbers 54439 to 55296)). The nucleotide sequence of the *purR* gene and the amino acid sequence encoded by the gene are shown in SEQ ID NOS: 5 and 6 of Sequence Listing (Japanese Patent Laid-open No. 2004-242610).

Primers used for PCR for obtaining the *purR* gene may be any of those enabling amplification of a part or full length of the *purR* gene, and specific examples include oligonucleotides having the nucleotide sequences shown in SEQ ID NO: 17 (GAAGTTGATGATCAAAA) and SEQ ID NO: 18 (ACATATTGTTGACGATAAT).

The *purR* gene used for preparation of the disrupted-type gene does not necessarily need to be the full length *purR* gene, and it may have a length required to cause gene disruption. Moreover, the microorganism used for obtaining each gene is not particularly limited, so long as a microorganism in which the gene has a homology to the *purR* gene of the *Bacillus* bacterium used for construction of a gene-disrupted strain to such an extent that they cause homologous recombination. However, it is usually preferable to use the gene originating in the same *Bacillus* bacterium as the objective *Bacillus* bacterium.

Examples of the marker gene described above include drug resistance genes such as a spectinomycin resistance gene, kanamycin resistance gene and tetracycline resistance gene. The spectinomycin resistance gene of *Enterococcus faecalis* can be obtained by preparing the plasmid pDG1726 from the *Escherichia coli* ECE101 strain commercially available from the *Bacillus* Genetic Stock Center (BGSC) and removing the resistance gene as a cassette from the plasmid. The erythromycin resistance gene of *Staphylococcus aureus* can be obtained by preparing the plasmid pDG646 from the *Escherichia coli* ECE91 strain commercially available from the *Bacillus* Genetic Stock Center (BGSC) and removing the resistance gene as a cassette from the plasmid. The kanamycin resistance gene derived from *Streptococcus faecalis* can be obtained by preparing the plasmid pDG783 from the *Escherichia coli* ECE94 strain commercially available from the *Bacillus* Genetic Stock Center (BGSC) and removing the resistance gene as a cassette from the plasmid. Furthermore, the chloramphenicol resistance gene of *Staphylococcus aureus* can be obtained by preparing the plasmid pC194 from the *Bacillus subtilis* 1E17 strain commercially available from the *Bacillus* Genetic Stock Center (BGSC) and performing PCR using that plasmid as a template to amplify the plasmid. The tetracycline resistance gene of *Streptococcus agalactiae* can be obtained by preparing the plasmid pDG1513 from the *Escherichia coli* ECE99 strain, and removing the plasmid as a cassette from the plasmid (Gene, 1995, 167:335-336).

When a drug resistance gene is used as the marker gene, a *purR* gene-disrupted strain can be obtained by inserting the drug resistance gene into the *purR* gene in a plasmid at an appropriate site, transforming a microorganism with the obtained plasmid and selecting a transformant that has become drug resistant. Disruption of the *purR* gene on the chromosome can be confirmed by analyzing the *purR* gene or the marker gene on the chromosome using Southern blotting or PCR. Incorporation of the aforementioned spectinomycin resistance gene, erythromycin resistance gene or kanamycin resistance gene into a chromosomal DNA can be confirmed by PCR using primers which can amplify these genes.

It is also known that expression of the purine operon is regulated by the terminator-antiterminator sequence locating downstream of the promoter (henceforth referred to as attenuator sequence) (Ebbole, D.J. and Zalkin, H., J. Biol. Chem., 1987, 262, 8274-8287; Ebbole D.J. and Zalkin H., J. Biol. Chem., 1988, 263, 10894-10902; Ebbole, D.J. and Zalkin, H., J. Bacteriol., 1989, 171, 2136-2141). Therefore, expression amount of the purine operon can be increased by deleting the attenuator sequence. Deletion of the attenuator sequence can be attained by the same method as that for disruption of *purR.*

In order to further increase transcription of the purine operon, the methods described above may be combined. For example, the *purR* gene may be disrupted, and further, the purine operon from which the attenuator sequence is deleted may be amplified using a plasmid or multiple copies of such a purine operon may be introduced into a chromosome.

The activities of an enzyme involved in purine biosynthesis can also be enhanced by desensitizing the enzyme which participates in the purine biosynthesis to regulation thereof, for example, according to the aforementioned method for desensitizing an enzyme to feedback inhibition (WO99/03988).

Furthermore, the ability to produce purine-derived substance can also be enhanced by attenuating uptake of a purine-derived substances into cells. For example, the uptake of purine nucleosides by the cells can be attenuated by blocking a reaction involved in the uptake of the purine nucleosides by the cells. An example of the reaction involved in the uptake of the purine nucleosides by the cells is includes reactions catalyzed by nucleoside permeases.

Furthermore, when a purine nucleoside is produced, activity of an enzyme which decomposes the purine nucleoside may be decreased in order to enhance the ability to produce purine nucleoside. An examples of such an enzyme includes purine nucleoside phosphorylase.

Purine nucleotides biosynthesized from PRPP by enzymes involved in purine biosynthesis are dephosphorylated and thereby converted into a purine nucleoside. To efficiently cause accumulation of a purine nucleoside, it is preferable to reduce an activity of purine nucleoside phosphorylases, which further degrade purine nucleosides into hypoxanthine or the like. That is, it is preferable to attenuate or eliminate an activity of a purine nucleoside phosphorylase that employs purine nucleosides such as inosine, as a substrate, .

Specifically, the purine nucleoside phosphorylase activity can be decreased by disrupting the deoD and *pupG* genes encoding purine nucleoside phosphorylase in *Bacillus* bacteria. The *Bacillus* bacterium of the present invention may be modified by disrupting one or both of the deoD and *pupG* genes . As the deoD and the *pupG* genes, for example, those genes derived from *Bacillus* bacteria (deoD: Genbank Accession No. NC_000964 (SEQ ID NO: 7), *pupG:* Genbank Accession No. NC_000964 (SEQ ID NO: 9)) can be used, and a gene-disrupted strain can be obtained in the same manner as that used for the aforementioned disruption of the *purR* gene.

The ability to produce a purine-derived substance may also enhanced by decreasing the activity of succinyl-AMP synthase. An example of the gene encoding succinyl-AMP synthase includes the *purA* gene. A example of the *purA* gene includes, for example, those having the nucleotide sequence registered as GenBank Accession No. NC_000964 (coding region corresponds to the nucleotide numbers 4153460 to 4155749 of the complementary strand, SEQ ID NO: 11).

The ability to produce a purine-derived substance may also be enhanced by decreasing an activity of inosine monophosphate (IMP) dehydrogenase. An example of the gene encoding IMP dehydrogenase includes the *guaB* gene. An examples of the *guaB* gene includes, for example, those having the nucleotide sequence registered as GenBank Accession No. NC_000964 (coding region corresponds to the nucleotide numbers 15913 to 17376, SEQ ID NO: 13).

Furthermore, the ability to produce a purine-derived substance, may also be enhanced by decreasing an activity of fructose bisphosphatase. An example of the gene encoding fructose bisphosphatase includes the fbp gene. An examples of the fbp gene includes, for example, those having the nucleotide sequence registered as GenBank Accession No. NC_000964 (coding region corresponds to the nucleotide numbers 4127053 to 4129065, SEQ ID NO: 15).

Moreover, as a method for enhancing an ability to produce purine-derived substance, amplification of a gene encoding a protein having an activity of secreting a purine-derived substance may be contemplated. An example of a bacterium in which such a gene has been amplified includes a *Bacillus* bacterium in which the rhtA gene is amplified (Japanese Patent Laid-open No. 2003-219876).

The *purR, deoD, pupG, purA, guaB* and *fbp* genes to be disrupted as described above, and the *prs* gene of which expression is to be enhanced may be conservative variants, for example, DNAs encoding proteins having amino acid sequences of SEQ ID NOS: 6, 8, 10, 12, 14, 16 and 4 which includes substitutions, deletions, insertions, additions or inversions of one or several amino acid residues, respectively, and having activity of the purine repressor, purine nucleoside phosphorylase, succinyl-AMP synthase, IMP dehydrogenase, fructose bisphosphatase or phosphoribosyl pyrophosphate synthetase, respectively. Number meant by the aforementioned term "several" is, for example, 2 to 50, preferably 2 to 30, more preferably 2 to 10.

Such change of amino acid sequences as described above is usually conservative change which maintains activities of proteins. Examples of conservative amino acid substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Specific examples of conservative variants of the *purR, deoD, pupG, purA, guaB* and *fbp* genes and the *prs* gene described above include DNAs showing a homology of, for example, 70% or more, preferably 80% or more, more preferably 90% or more, particularly preferably 95% or more, to DNAs having the nucleotide sequences of SEQ ID NOS: 5, 7, 9, 11, 13, 15 and 3, respectively. More specifically, the examples of the conservative variants include DNAs that are able to hybridize with DNAs having nucleotide sequences complementary to the nucleotide sequences of SEQ ID NOS: 5, 7, 9, 11, 13, 15 and 3 under stringent conditions. An example of the stringent conditions includes conditions of washing at 60°C and salt concentrations of 1×SSC, 0.1% SDS, preferably 0.1×SSC, 0.1% SDS, once or more times, preferably twice or three times.

Homology of DNAs can be evaluated by BLAST search, FASTA search, the calculation method of Crustal W, and so forth.

BLAST (basic local alignment search tool) is a heuristic search algorithm used by the programs blastp, blastn, blastx, megablast, tblastn and tblastx, and the results obtained by these programs are considered significant on the basis of use of the statistical method of Karlin, Samuel, and Stephen F. Altschul ("Methods for assessing the statistical significance of molecular sequence features by using general scoring schemes", Proc. Natl. Acad. Sci. USA, 1990, 87:2264-68; "Applications and statistics for multiple high-scoring segments in molecular sequences", Proc. Natl. Acad. Sci. USA, 1993, 90:5873-7). The FASTA search method was described by W.R. Pearson ("Rapid and Sensitive Sequence Comparison with FASTP and FASTA", Methods in Enzymology, 1990 183:63-98). The Clustal W method is described by Thompson J.D., Higgins D.G., and Gibson T.J. ("CLUSTAL W: Improving the sensitivity of progressive multiple sequence alignment through sequence weighting, position-specific gap penalties and weight matrix choice", Nucleic Acids Res., 1994, 22:4673-4680).

Moreover, DNA used for preparation of a disrupted-type gene may also be a conservative variant of *purR, deoD, pupG, purA* or *guaB* gene.

Incorporation of a target gene into the chromosomal DNA of *Bacillus* bacterium can be performed in the same manner as that for the gene encoding transaldolase described later.

### (2) Modification for decreasing enzymatic activity of transaldolase

The *Bacillus* bacterium of the present invention can be obtained by modifying a strain having the ability to produce a purine-derived substance such as those described above so that the enzymatic activity of transaldolase is decreased. The order of modification is not limited, and after modifying a bacterium so that the enzymatic activity of transaldolase thereof is decreased, purine nucleotide producing ability may be imparted to the bacterium.

Transaldolase is an enzyme catalyzing the reaction reversibly generating D-erythrose-4-phosphate and D-fructose-6-phosphate from sedoheptulose-7-phosphate and D-glyceraldehyde-3-phosphate, and this reaction is one of the reactions of the pentose phosphate pathway. The "pentose phosphate pathway" means the pathway in which glucose taken up into cells is phosphorylated by glucose kinase to biosynthesize glucose-6-phosphate, and glucose-6-phosphate is oxidatively converted into ribose-5-phosphate, and the pathway consisting of reversible process for interconversion of phosphates of triose, tetrolose, pentose, hexose and heptose by the actions of epimerase, transketolase (EC: 2.2.1.1) and transaldolase (EC: 2.2.1.2).

The enzymatic activity of transaldolase can be measured by the following method. For example, the activity can be measured by converting generated D-glyceraldehyde-3-phosphate into hydroxyacetone phosphate with triosephosphate isomerase and measuring hydroxyacetone phosphate using glycerol-3-phosphate dehydrogenase and NADH (Ochoa, T., and Horecker, B.L., 1966, Methods Enzymol., 9, 499-505).

Such modification that the enzymatic activity of transaldolase is decreased can be attained by, for example, as explained above for disruption of the *purR* gene, substituting a corresponding gene which does not function normally (e.g., disrupted-type gene obtained by inserting a marker gene such as drug resistance gene into the transaldolase gene) for the transaldolase gene on the chromosome by homologous recombination. Furthermore, as described for the *purR* gene, a mutation for reducing intracellular enzymatic activity of transaldolase may be introduced into the transaldolase gene on the chromosome by conventional mutagenesis methods.

Examples of transaldolase of *Bacillus subtilis* include a protein consisting of 212 amino acid residues shown in SEQ ID NO: 2, and a gene encoding the protein, preferably a gene having the nucleotide sequence of SEQ ID NO: 1 (*ywjH* gene, Genbank Accession No. NC_000964), can be used for the modification. The *ywjH* gene exists at about 325° on the *Bacillus subtilis* chromosome.

The gene encoding transaldolase may also be a conservative variant of the *ywjH* gene like the aforementioned genes. Specifically, examples include a DNA encoding a protein having the amino acid sequence of SEQ ID NO: 1 including substitutions, deletions, insertions, additions or inversions of one or several amino acid residues and having the enzymatic activity of transaldolase. Examples further include a DNA encoding a protein showing a homology of 50% or more, preferably 70% or more, more preferably 80% more, particularly preferably 90% or more, most preferably 95% or more, to the amino acid sequence shown in SEQ ID NO: 2, and having the enzymatic activity of transaldolase. More specifically, examples include a DNA which is able to hybridize with a DNA having the nucleotide sequence of SEQ ID NO: 1 under stringent conditions. The stringent conditions include conditions of washing at 60°C and salt concentrations of 1×SSC, 0.1% SDS, preferably 60°C, 0.1×SSC, 0.1% SDS, once or more times, preferably twice or three times.

Such a DNA encoding a protein substantially identical to transaldolase as described above can be obtained by, for example, modifying a nucleotide sequence encoding such an enzyme so that the amino acid residue of a specific part is substituted, deleted, inserted, added or inversed by the site-specific mitagenesis. Such a modified DNA as described above may also be obtained by a conventionally known mutagenesis treatment. Examples of the mutagenesis treatment include an in vitro treatment of DNA before mutagenesis treatment with hydroxylamine, and a treatment of a microorganism such as *Escherichia* bacterium containing DNA before mutagenesis treatment with ultraviolet irradiation or a mutagen used for conventional mutagenesis treatment such as N-methyl-N'-nitro-N-nitrosoguanidine (NTG) or nitrous acid.

The target gene can be obtained by, for example, PCR method (polymerase chain reaction, White, T.J. et al., Trends Genet., 1989, 5, 185-189) using a chromosomal DNA of a *Bacillus* bacterium as a template and oligonucleotides prepared based on nucleotide sequence of target gene as primers. The chromosomal DNA can be prepared from a bacterium serving as a DNA donor by, for example, the method of Saito and Miura (H. Saito and K. Miura, Biochem. Biophys. Acta, 1963, 72, 619-629; Text for Bioengineering Experiments, Edited by the Society for Bioscience and Bioengineering, Japan, pp.97-98, Baifukan, 1992) or the like. The primers for PCR can be prepared based on a known gene sequence of a *Bacillus* bacterium, or based on information on a region conserved among genes of other bacteria of which sequences are known.

Examples of the vector for incorporating a target gene into a chromosomal DNA of *Bacillus* bacterium include a vector having a temperature sensitive replication origin, such as pHV1248 (Prtit, M.-A., et. al., J. Bacteriol., 1990, 172, 6736-6740), vectors for E. *coli* such as pHSG398 (Takara Shuzo) and pBluescript SK- (Stratagene), and so forth.

In order to ligate an objective gene and a vector carrying a marker which functions in *Bacillus* bacteria to prepare a recombinant DNA, the vector is digested with a restriction enzyme matching the end of the objective gene. The ligation is usually performed by using a ligase such as T4 DNA ligase.

To introduce a recombinant DNA prepared as described above into a *Bacillus* bacterium, any of known transformation methods that have hitherto been reported can be employed. Examples include, for instance, a method of preparing competent cells from cells which are at the growth phase followed by introducing the DNA thereinto, (Dubunau D. and Davidoff-Abelson, R., J. Mol. Biol., 1971, 56, 209-221) and a method of making host cells into protoplasts or spheroplasts, which can easily take up recombinant DNA, followed by introducing the recombinant DNA into the DNA-acceptor cells (Chang, S. and Choen, S.N., Molec. Gen. Genet., 1979, 168, 111-115).

### <2> Method for producing a purine-derived substance

The *Bacillus* bacterium of the present invention efficiently produces a purine-derived substance. Therefore, by culturing the *Bacillus* bacterium of the present invention in an appropriate medium, a purine-derived substance, such as purine nucleoside and purine nucleotide can be produced and accumulated in cells of the bacterium or the medium.

As for the medium used for the present invention, culture can be performed in a conventional manner using a conventinal medium containing a carbon source, nitrogen source and mineral salts as well as organic trace nutrients such as amino acids and vitamins, as required. Either a synthetic medium or a natural medium may be used. Any kinds of carbon source and nitrogen source may be used so long as they can be utilized by a strain to be cultured.

As the carbon source, sugars such as glucose, fructose, sucrose, maltose, mannose, galactose, arabinose, xylose, trehalose, ribose, starch hydrolysates and molasses, and alcohols such as glycerol and mannitol are used, and organic acids such as gluconic acid, acetic acid, citric acid, maleic acid, fumaric acid and succinic acid can also be used independently or in combination with other carbon sources.

As the nitrogen source, ammonia, ammonium salts such as ammonium sulfate, ammonium carbonate, ammonium chloride, ammonium phosphate and ammonium acetate, nitric acid salts, organic nitrogen such as soybean hydrolysate, and so forth are used.

As the organic trace nutrients, amino acids, vitamins, fatty acids, nucleic acids, those containing these substances such as peptone, casamino acid, yeast extract and soybean protein decomposition product and so forth are used. When an auxotrophic mutant strain that requires an amino acid or the like for growth is used, it is necessary to supplement the required nutrient.

As the mineral salts, phosphoric acid salts, magnesium salts, calcium salts, iron salts, manganese salts and so forth are used.

Although the culture conditions may vary depending on the type of *Bacillus* bacterium to be used, *Bacillus subtilis,* for example, is cultured as aeration culture, while the fermentation temperature is controlled to be 20 to 50°C, and pH to be 4 to 9. When pH falls during the culture, the medium is neutralized with an alkali such as ammonia gas. A purine nucleoside is accumulated in the medium after about 40 hours to 3 days of culture in such a manner as described above.

After completion of the culture, inosine accumulated in the medium may be collected in a conventional manner. For example, it can be isolated by precipitation, ion exchange chromatography and so forth.

Furthermore, if the microorganism used for the present invention lacks a gene encoding a nucleosidase or nucleotidase, a corresponding nucleoside or nucleotide can be accumulated. Furthermore, if inosine auxotrophy is imparted, a precursor or relevant substances involved in the biosynthesis pathway thereof can be accumulated.

Furthermore, by reacting inosine or guanosine prepared by the method of the present invention with purine nucleoside phosphorylase or phosphoribosyltransferase , 5'-inosinic acid or 5'-guanylic acid can be obtained.

Moreover, it is also possible to phosphorylate the purine nucleoside produced using the microorganism of the present invention by reacting phosphotransferase on the purine nucleoside to produce a purine nucleotide (nucleoside 5'-phosphoric acid ester) (Japanese Patent Laid-open No. 2000-295996). For example, the method for producing a purine nucleotide using an *Escherichia* bacterium into which a gene encoding inosine guanosine kinase of *Escherichia coli* is introduced (WO91/08286), and the method for producing a purine nucleotide using *Corynebacterium ammoniagenes* into which a gene encoding inosine guanosine kinase of *Exiguobacterium acetylicum* is introduced (WO96/30501) can be used.

Moreover, it is also possible to produce a purine nucleotide (nucleoside 5'-phosphoric acid ester) by reacting the purine nucleoside produced by using the microorganism of the present invention with a phosphate donor selected from the group consisting of polyphosphoric acid, phenyl phosphate and carbamyl phosphate, and a microorganism having an ability to produce a nucleoside 5'-phosphoric acid ester or acid phosphatase (EC 3.1.3.2). Although the microorganism having an ability to produce a nucleoside 5'-phosphoric acid ester is not particularly limited so long as a microorganism having an ability to convert a purine nucleoside into a purine nucleotide is chosen, examples include, for example, the microorganism disclosed in International Patent Publication WO96/37603.

Moreover, *Escherichia blattae* JCM 1650, *Serratia ficaria* ATCC 33105, *Klebsiella planticola* IFO 14939 (ATCC 33531), *Klebsiella pneumoniae* IFO 3318 (ATCC 8724), *Klebsiella terrigena* IFO 14941 (ATCC 33257), *Morganella morganii* IFO 3168, *Enterobacter aerogenes* IFO 12010, *Enterobacter aerogenes* IFO 13534 (ATCC 13048), *Chromobacterium fluviatile* IAM 13652, *Chromobacterium violaceum* IFO 12614, *Cedecea lapagei* JCM 1684, *Cedecea davisiae* JCM 1685, *Cedecea neteri* JCM 5909, and so forth disclosed in Japanese Patent Laid-open No. 07-231793 can also be used.

As the acid phosphatase, for example, the acid phosphatase disclosed in Japanese Patent Laid-open No. 2002-000289 can be used, and an acid phosphatase of which affinity to a nucleoside is increased (Japanese Patent Laid-open No. 10-201481), a mutant acid phosphatase of which nucleotidase activity is decreased (WO96/37603), a mutant acid phosphatase of which phosphoric acid ester hydrolysis activity is decreased (Japanese Patent Laid-open No. 2001-245676) and so forth can be more preferably used.

It is also possible to obtain a purine nucleotide by chemically phosphorylating a purine nucleoside produced using the microorganism of the present invention (Bulletin of the Chemical Society of Japan, 42, 3505). Moreover, a method of obtaining GMP by coupling the microorganism having an XMP-producing ability of the present invention and XMP aminase activity using the ATP-regenerating system of a microorganism, and a method of obtaining IMP by coupling inosine kinase (Biosci. Biotech. Biochem., 51, 840 (1997); Japanese Patent Laid-open No. 63-230094) can also be used.

Inosine, guanosine or purine nucleoside prepared by the method of the present invention used for the aforementioned preparation of purine nucleotide may be a purified product, or purine nucleoside fermentation broth, or a crude product containing a purine nucleoside.

### Examples

Hereafter, the present invention will be more specifically explained with reference to examples.

### Example 1

### <Construction of bacterial strain deficient in pupG and deoD genes encoding purine nucleoside phosphorylases>

A strain deficient in the purine nucleoside phosphorylase gene (*deoD*) was constructed from the recombinant strain KMBS310 as described below. The KMBS310 strain (Japanese Patent Application No. 2005-280186), which is derived from *Bacillus subtilis (B. subtilis* 168 Marburg strain, ATCC 6051), is deficient in the purine operon repressor gene *(purR),* succinyl-AMP synthase gene (*purA*) and purine nucleoside phosphorylase gene *(pupG),* and has attenuated IMP dehydrogenase gene *(guaB),* wherein the purine operon promoter region and SD sequence of the PRPP synthetase gene *(prs)* was modified (Japanese Patent Application No. 2005-280186). Expression of the purine operon and the PRPP synthetase gene was enhanced by the modifications of the promoter region and the SD sequence, respectively.

A genome DNA prepared from the KMBS16 strain (purR::spc purA::erm deoD::kan, Japanese Patent Laid-open No. 2004-242610) by the method of Fouet and Sonenshein (J. Bacteriol., 1990, 172, 835-844) was used to transform competent cells of the *B. subtilis* 168 Marburg strain prepared by the method of Dubnau and Davidoff-Abelson (J. Mol. Biol., 1971, 56, 209-221), and colonies grown on an LB agar plate containing 5 µg/ml of kanamycin were obtained. Colonies which did not became spectinomycin-resistant nor erythromycin-resistant were selected from the obtained colonies, and one strain among them was designated KMBS5 (deoD::kan).

A genomic DNA prepared from KMBS5 by the method of Fouet and Sonenshein (J. Bacteriol., 1990, 172, 835-844) was used to transform competent cells of the KMBS310 strain prepared by the method of Dubnau and Davidoff-Abelson (J. Mol. Biol., 1971, 56, 209-221), and colonies grown on an LB agar plate containing 5 µg/ml of kanamycin and 20 µg/ml of guanine were obtained. Several colonies were selected from the colonies obtained as described above, and one of transformants for which it could be confirmed that deoD::kan substituted for the wild-type deoD gene, and all the mutations derived from KMBS310 were not replaced with wild-type sequences was designated KMBS321.

### Example 2

### <Construction of bacterial strain deficient in either one or both of fbp gene encoding fructose bisphosphatase and ywjH gene encoding transaldolase, culture and evaluation thereof>

### (1) Preparation of fbp gene-deficient strain

A strain deficient in the fructose bisphosphatase gene (*fbp*) was constructed from the aforementioned recombinant strain KMBS321 as described below. The KMBS321 strain, which is derived from *Bacillus subtilis (B. subtilis* 168 Marburg strain, ATCC 6051), is deficient in the purine operon repressor gene *(purR),* succinyl-AMP synthase gene (*purA*) and purine nucleoside phosphorylase gene *(pupG),* and has attenuated IMP dehydrogenase gene *(guaB),* wherein modified purine operon promoter region and SD sequence of the PRPP synthetase gene *(prs)* is modified.

### (i) Amplification of fbp upstream region by PCR

28-mer and 50-mer PCR primers having the following nucleotide sequences were prepared based on the information of a gene data bank (GenBank Accession Nos. NC_000964 and V01277).
TTCCCTTAGGGTTATTTTCGTTTCAAAA (SEQ ID NO: 19)
cgtttgttgaactaatgggtgctTTTATGAGCATGTGCATGATAAGGTGA (SEQ ID NO: 20, the nucleotides indicated with small letters correspond to the promoter upstream region of the chloramphenicol resistance gene (cat) cloned in pC194)

PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *fbp* gene 5' end region and about 1350 bp upstream region.

### (ii) Amplification of fbp downstream region by PCR

50-mer and 27-mer PCR primers having the following nucleotide sequences were prepared based on the information of a gene data bank (GenBank Accession Nos. NC_000964 and V01277).
acagctccagatccatatccttcttTTTTAGAGAGTTTGCGGGAGTATCG (SEQ ID NO: 21, the nucleotides indicated with small letters correspond to a downstream region of structural gene of the chloramphenicol resistance gene (*cat*) cloned in pC194)
TAAAGGTTTTTCGGGATAAGATTGAAA (SEQ ID NO: 22)
PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a fbp gene 3' end region and about 1770 bp downstream region.

### (iii) Amplification of cat gene by PCR

50-mer PCR primers having the following nucleotide sequences were prepared based on the information of a gene data bank (GenBank Accession Nos. V01277 and NC_000964).
tcaccttatcatgcacatgctcataaaAGCACCCATTAGTTCAACAAACG (SEQ ID NO: 23, the nucleotides indicated with small letters correspond to the sequence of 5' end region of the fbp gene, and they were designed so as to be complementary to the 3' end region of SEQ ID NO: 20)
cgatactcccgcaaactctctaaaaAAGAAGGATATGGATCTGGAGCTGT (SEQ ID NO: 24, the nucleotides indicated with small letters correspond to the sequence of 3' end region of the fbp gene, and they were designed so as to be complementary to the 3' end region of SEQ ID NO: 21)
PCR (98°C for 10 second, 55°C for 30 seconds, 72°C for 1.5 minutes, 30 cycles, Gene Amp PCR System Model 9600, Perkin-Elmer) was performed by using the plasmid pC194 carrying the chloramphenicol resistance gene (cat) as a template and the aforementioned primers to obtain an amplification fragment of about 980 bp containing the cat gene.

### (iv) Amplification of fragment comprising fbp region inserted with cat gene by recombinant PCR

The DNA fragments amplified in (i) to (iii) described above were purified using MicroSpin Column S-400 (Amersham Pharmacia Biotech), and then a mixture of them in appropriate amounts was used as a template together with the sequences of SEQ ID NOS: 19 and 22 to perform PCR (98°C for 10 second, 55°C for 30 seconds, 72°C for 4.5 minutes, 30 cycles, Gene Amp PCR System Model 9600, Perkin-Elmer) and thereby obtain a fragment comprising the fbp region inserted with the cat gene.

### (v) Preparation of fbp-disrupted inosine-producing strain

The DNA fragment comprising the *fbp* region into which the cat gene (fbp::cat) obtained in (iv) had been inserted was subjected to agarose gel electrophoresis, and the target fragment was extracted from the gel. The DNA fragment purified as described above was used to transform competent cells of the *B. subtilis* KMBS321 strain prepared by the method of Dubnau and Davidoff-Abelson (J. Mol. Biol., 1971, 56, 209-221), and colonies grown on an LB agar plate containing 2.5 µg/ml of chloramphenicol and 20 µg/ml of guanine were obtained. Chromosomal DNAs were prepared from these colonies, strains in which fbp region on a chromosome was replaced with the fbp region of which internal sequence was replaced with the chloramphenicol resistance gene (fbp::cat) by double recombination were identified by the PCR method described in (iv), and one of these strains was designated TABS133.

### (2) Preparation of ywjH gene-deficient strain

A strain deficient in the transaldolase gene (*ywjH*) was constructed from the aforementioned recombinant strain KMBS321 as described below. The KMBS321 strain (Japanese Patent Application No. 2005-280186), which is derived from *Bacillus subtilis (B. subtilis* 168 Marburg strain, ATCC 6051), is deficient in the purine operon repressor gene (purR), succinyl-AMP synthase gene (purA) and purine nucleoside phosphorylase gene (pupG), and has attenuated IMP dehydrogenase gene (*guaB*), wherein the purine operon promoter region and SD sequence of the PRPP synthetase gene *(prs),* and deficient in the purine nucleoside phosphorylase gene (deoD) is modified.

### (i) Amplification of ywjH upstream region by PCR

28-mer and 50-mer PCR primers having the following nucleotide sequences were prepared based on the information of a gene data bank (GenBank Accession Nos. NC_000964 and V01277).
ATGGACGGAATCGAAATCTTAAAACGGA (SEQ ID NO: 25) cgtttgttgaactaatgggtgctttAGAATTCCTAATTCATTCGCTTCTC (SEQ ID NO: 26, the nucleotides indicated with small letters correspond to the promoter upstream region of the chloramphenicol resistance gene (cat) cloned in pC194)
PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *ywjH* gene 5' end region and about 1420 bp upstream region.

### (ii) Amplification of ywjH downstream region by PCR

50-mer and 28-mer PCR primers having the following nucleotide sequences were prepared based on the information of a gene data bank (GenBank Accession Nos. NC_000964 and V01277).
acagctccagatccatatccttctTTGACCTGATTTCAGAAGTTAAACAG (SEQ ID NO: 27, the nucleotides indicated with small letters correspond to a downstream region of structural gene of the chloramphenicol resistance gene (cat) cloned in pC194) GGATGACGTTATCGATAATGACTTCCTT (SEQ ID NO: 28)
PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *ywjH* gene 3' end region and about 1370 bp downstream region.

### (iii) Amplification of cat gene by PCR

50-mer PCR primers having the following nucleotide sequences were prepared based on the information of a gene data bank (GenBank Accession Nos. V01277 and NC_000964).
gagaagcgaatgaattaggaattctAAAGCACCCATTAGTTCAACAAACG (SEQ ID NO: 29, the nucleotides indicated with small letters correspond to the sequence of 5' end region of the *ywjH* gene, and they were designed so as to be complementary to the 3' end region of SEQ ID NO: 26)
ctgtttaacttctgaaatcaggtcaaAGAAGGATATGGATCTGGAGCTGT (SEQ ID NO: 30, the nucleotides indicated with small letters correspond to the sequence of 3' end region of the *ywjH* gene, and they were designed so as to be complementary to the 3' end region of SEQ ID NO: 27)

PCR (98°C for 10 second, 55°C for 30 seconds, 72°C for 1.5 minutes, 30 cycles, Gene Amp PCR System Model 9600, Perkin-Elmer) was performed using the plasmid pC194 carrying the chloramphenicol resistance gene *(cat)* as a template and the aforementioned primers to obtain an amplification fragment of about 980 bp containing the *cat* gene.

### (iv) Amplification of fragment comprising ywjH region inserted with cat gene by recombinant PCR

The DNA fragments amplified in (i) to (iii) mentioned above were purified using MicroSpin Column S-400 (Amersham Pharmacia Biotech), and then a mixture of them in appropriate amounts was used as a template together with the sequences of SEQ ID NOS: 25 and 28 to perform PCR (98°C for 10 second, 55°C for 30 seconds, 72°C for 4 minutes, 30 cycles, Gene Amp PCR System Model 9600, Perkin-Elmer) and thereby obtain a fragment comprising the *ywjH* region inserted with the cat gene.

### (v) Preparation of ywjH-disrupted inosine-producing strain

The DNA fragment comprising the *ywjH* region into which the cat gene (ywjH::cat) obtained in (iv) had been instersed was subjected to agarose gel electrophoresis, and the target fragment was extracted from the gel. The DNA fragment purified as described above was used to transform competent cells of the *B. subtilis* KMBS321 strain prepared by the method of Dubnau and Davidoff-Abelson (J. Mol. Biol., 1971, 56, 209-221), and colonies grown on an LB agar plate containing 2.5 µg/ml of chloramphenicol and 20 µg/ml of guanine were obtained. Chromosomal DNAs were prepared from these colonies, strains in which *ywjH* region on a chromosome was replaced with the *ywjH* region of which internal sequence was replaced with the chloramphenicol resistance gene (ywjH::cat) by double recombination were identified by the PCR method described in (iv), and one of these strains was designated TABS135.

### (3) Construction of fbp and ywjH double-deficient strain

A strain deficient in the transaldolase gene (*ywjH*) was constructed from the recombinant strain TABS133 as described below. The TABS133 strain, which is derived from *Bacillus subtilis (B. subtilis* 168 Marburg strain, ATCC 6051), is deficient in the purine operon repressor gene *(purR),* succinyl-AMP synthase gene (purA), purine nucleoside phosphorylase gene (*pupG*) and fructose bisphosphatase gene *(fbp),* and has attenuated IMP dehydrogenase gene *(guaB),* wherein the purine operon promoter region and SD sequence of the PRPP synthetase gene *(prs)* is modified.

### (i) Amplification of ywjH upstream region by PCR

26-mer and 50-mer PCR primers having the following nucleotide sequences were prepared based on the information of a gene data bank (GenBank Accession Nos. NC_000964 and M29725).
TAAAGCGTGATAGACATACAGTGCTG (SEQ ID NO: 31) cattgcaagacttttttcaccaagcAGAATTCCTAATTCATTCGCTTCTC (SEQ ID NO: 32, the nucleotides indicated with small letters correspond to the promoter upstream region of the tetracycline resistance gene (tet) cloned in pDG1513)
PCR (98°C for 10 second, 55°C for 30 seconds, 72°C for 2.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *ywjH* gene 5' end region and about 2250 bp upstream region.

### (ii) Amplification of ywjH downstream region by PCR

50-mer and 26-mer PCR primers having the following nucleotide sequences were prepared based on the information of a gene data bank (GenBank Accession Nos. NC_000964 and M29725).
gagagagttcaaaattgatcctttTTGACCTGATTTCAGAAGTTAAACAG (SEQ ID NO: 33, the nucleotides indicated with small letters correspond to a downstream region of structural gene of the tetracycline resistance gene (tet) cloned in pDG1513)
TTGCATATACATGTCAGGAGCATTCA (SEQ ID NO: 34)
PCR (98°C for 10 second, 55°C for 30 seconds, 72°C for 2.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *ywjH* gene 3' end region and about 2280 bp downstream region.

### (iii) Amplification of tet gene by PCR

50-mer PCR primers having the following nucleotide sequences were prepared based on the information of a gene data bank (GenBank Accession Nos. M29725 and NC_000964).
gagaagcgaatgaattaggaattctGCTTGGTGAAAAAAGTCTTGCAATG (SEQ ID NO: 35, the nucleotides indicated with small letters correspond to the sequence of 5' end region of the *ywjH* gene, and they were designed so as to be complementary to the 3' end region of SEQ ID NO: 32)
ctgtttaacttctgaaatcaggtcaaAAAGGATCAATTTTGAACTCTCTC (SEQ ID NO: 36, the nucleotides indicated with small letters correspond to the sequence of 3' end region of the *ywjH* gene, and they were designed so as to be complementary to the 3' end region of SEQ ID NO: 33)
PCR (98°C for 10 second, 55°C for 30 seconds, 72°C for 2.5 minutes, 30 cycles, Gene Amp PCR System Model 9600, Perkin-Elmer) was performed using the plasmid pDG1513 carrying the tetracycline resistance gene (tet) as a template and the aforementioned primers to obtain an amplification fragment of about 2070 bp containing the *cat* gene.

### (iv) Amplification of fragment comprising ywjH region inserted with tet gene by recombinant PCR

The DNA fragments amplified in (i) to (iii) mentioned above were purified by using MicroSpin Column S-400 (Amersham Pharmacia Biotech), and then a mixture of them in appropriate amounts was used as a template together with the sequences of SEQ ID NOS: 31 and 34 to perform PCR (98°C for 10 second, 55°C for 30 seconds, 72°C for 7 minutes, 30 cycles, Gene Amp PCR System Model 9600, Perkin-Elmer) and thereby obtain a fragment comprising the *ywjH* region inserted with the tet gene.

### (v) Preparation of fbp and ywjH-disrupted inosine-producing strain

The DNA fragment comprising the *ywjH* region into which the tet gene (ywjH::tet) obtained in (iv) had been inserted was subjected to agarose gel electrophoresis, and the target fragment was extracted from the gel. The DNA fragment purified as described above was used to transform competent cells of the *B. subtilis* TABS133 strain prepared by the method of Dubnau and Davidoff-Abelson (J. Mol. Biol., 1971, 56, 209-221), and colonies grown on an LB agar plate containing 12.5 µg/ml of tetracycline and 20 µg/ml of guanine were obtained. Chromosomal DNAs were prepared from these colonies, strains in which ywjH region on a chromosome was replaced with the *ywjH* region of which internal sequence was replaced with the tetracycline resistance gene (ywjH::tet) by double recombination were identified by the PCR method described in (iv), and one of these strains was designated TABS174.

### (4) Production of purine nucleoside by inosine-producing strain deficient in either one or both of fbp gene and ywjH gene

The *fbp* gene-deficient strain TABS133, the *ywjH* gene-deficient strain TABS135, the both genes-deficient strain TABS174, and a control strain KMBS321 were each uniformly applied on an LB medium plate containing 20 mg/L of guanine, and cultured overnight at 34°C. The cells corresponding to 1/8 of the plate were inoculated into 20 ml of a fermentation medium contained in a 500-ml volume Sakaguchi flask, then 50 g/L of calcium carbonate was added to the medium, and the cells were cultured at 34°C with shaking. Seventy two hours after the start of the culture, the medium was sampled, and amounts of inosine and hypoxanthine contained in the medium were measured by known methods. The accumulated inosine amounts observed with the *fbp* gene-deficient strain TABS133 and the *ywjH* gene-deficient strain TABS135 were higher than that observed with the KMBS321 strain as a control strain. Furthermore, the accumulated inosine amount observed with the strain deficient in both genes was higher than that observed with the TABS133 strain or TABS135 strain.

| [Composition of fermentation medium] | |
|---|---|
| Glucose | 30 g/L |
| KH₂PO₄ | 1 g/L |
| NH₄Cl | 32 g/L |
| Mameno (T-N)* | 1.35 g/L |
| DL-Methionine | 0.4 g/L |
| L-Tryptophan | 0.02 g/L |
| Adenine | 0.1 g/L |
| Guanosine | 0.075 g/L |
| MgSO₄ | 0.4 g/L |
| FeSO₄ | 0.01 g/L |
| MnSO₄ | 0.01 g/L |
| Adekanol (antifoam) (adjusted to pH 7.0 with KOH) | 0.5 ml/L |
| Calcium Carbonate | 50 g/L |

| | |
|---|---|
| *: Soybean protein hydrolysate | |

**Table 1**

| *B. subtilis* strains | Inosine (g/L) |
|---|---|
| KMBS321 | 5.3 |
| TABS133 | 5.8 |
| TABS135 | 6.0 |
| TABS174 | 6.5 |

### [Explanation of Sequence Listing]

SEQ ID NO: 1: Nucleotide sequence of *ywjH* gene
SEQ ID NO: 2: Amino acid sequence of transaldolase
SEQ ID NO: 3: Nucleotide sequence of *prs* gene
SEQ ID NO: 4: Amino acid sequence of phosphoribosyl pyrophosphate synthetase
SEQ ID NO: 5: Nucleotide sequence of *purR* gene
SEQ ID NO: 6: Amino acid sequence of purine repressor
SEQ ID NO: 7: Nucleotide sequence of deoD gene
SEQ ID NO: 8: Amino acid sequence of deoD gene product (purine nucleoside phosphorylase)
SEQ ID NO: 9: Nucleotide sequence of *pupG* gene
SEQ ID NO: 10: Amino acid sequence of *pupG* gene product (purine nucleoside phosphorylase)
SEQ ID NO: 11: Nucleotide sequence of *purA* gene
SEQ ID NO: 12: Amino acid sequence of succinyl-AMP synthase
SEQ ID NO: 13: Nucleotide sequence of *guaB* gene
SEQ ID NO: 14: Amino acid sequence of IMP dehydrogenase
SEQ ID NO: 15: Nucleotide sequence of *fbp* gene
SEQ ID NO: 16: Amino acid sequence of fructose bisphosphatase
SEQ ID NO: 17: Primer for *purR* gene amplification
SEQ ID NO: 18: Primer for *purR* gene amplification
SEQ ID NO: 19: Primer for fbp gene upstream region amplification
SEQ ID NO: 20: Primer for fbp gene upstream region amplification
SEQ ID NO: 21: Primer for fbp gene downstream region amplification
SEQ ID NO: 22: Primer for *fbp* gene downstream region amplification
SEQ ID NO: 23: Primer for *cat* gene amplification
SEQ ID NO: 24: Primer for cat gene amplification
SEQ ID NO: 25: Primer for *ywjH* gene upstream region amplification
SEQ ID NO: 26: Primer for *ywjH* gene upstream region amplification
SEQ ID NO: 27: Primer for *ywjH* gene downstream region amplification
SEQ ID NO: 28: Primer for *ywjH* gene downstream region amplification
SEQ ID NO: 29: Primer for cat gene amplification
SEQ ID NO: 30: Primer for cat gene amplification
SEQ ID NO: 31: Primer for *ywjH* gene upstream region amplification
SEQ ID NO: 32: Primer for *ywjH* gene upstream region amplification
SEQ ID NO: 33: Primer for *ywjH* gene downstream region amplification
SEQ ID NO: 34: Primer for *ywjH* gene downstream region amplification
SEQ ID NO: 35: Primer for tet gene amplification
SEQ ID NO: 36: Primer for tet gene amplification

### Industrial Applicability

By using the *Bacillus* bacterium of the present invention, production efficiency of a purine nucleoside and/or a purine nucleotide can be improved.

## Claims

1. A bacterium belonging to the genus *Bacillus* which has an ability to produce a purine-derived substance, wherein the bacterium has been modified so that enzymatic activity of transaldolase is decreased.

2. The bacterium belonging to the genus *Bacillus* according to claim 1, wherein the purine-derived substance is a purine nucleoside selected from the group consisting of inosine, xanthosine, guanosine and adenosine.

3. The bacterium belonging to the genus *Bacillus* according to claim 1, wherein the purine-derived substance is a purine nucleotide selected from the group consisting of inosinic acid, xanthylic acid, guanylic acid and adenylic acid.

4. The bacterium belonging to the genus *Bacillus* according to any one of claims 1 to 3, wherein the transaldolase activity is decreased by disruption of a gene encoding transaldolase, or decreasing expression amount of the gene encoding transaldolase.

5. The bacterium belonging to the genus *Bacillus* according to claim 4, wherein the gene encoding transaldolase is a gene encoding a protein of the following (A) or (B):
(A) a protein comprising the amino acid sequence of SEQ ID NO: 1,
(B) a protein comprising the amino acid sequence of SEQ ID NO: 1 which includes substitutions, deletions, insertions, additions or inversions of one or several amino acid residues and has transaldolase activity.

6. The bacterium belonging to the genus *Bacillus* according to any one of claims 1 to 5, which has been further modified so that fructose bisphosphatase activity is decreased.

7. The bacterium belonging to the genus *Bacillus* according to any one of claims 1 to 6, which has been further modified so that phosphoribosyl pyrophosphate synthetase activity is increased.

8. The bacterium belonging to the genus *Bacillus* according to any one of claim 1 to 7, which has been further modified so that expression amount of the purine operon is increased.

9. The bacterium belonging to the genus *Bacillus* according to claim 8, wherein expression amount of the purine operon is increased by disruption of the *purR* gene, which is a gene encoding a repressor of the purine operon.

10. The bacterium belonging to the genus *Bacillus* according to any one of claims 1 to 9, which has been further modified so that purine nucleoside phosphorylase activity is decreased.

11. The bacterium belonging to the genus *Bacillus* according to any one of claim 1 to 10, which has been further modified so that IMP dehydrogenase activity is decreased.

12. The bacterium belonging to the genus *Bacillus* according to any one of claim 1 to 11, which is *Bacillus subtilis.*

13. A method for producing a purine-derived substance, which comprises
culturing the bacterium belonging to the genus *Bacillus* according to any one of claims 1 to 12 in a medium to cause accumulation of a purine-derived substance in cells of the bacterium or the medium, and
collecting the purine-derived substance from the cells or medium.

14. The method according to claim 13, wherein the purine-derived substance is a purine nucleoside or a purine nucleotide.

15. The method according to claim 14, wherein the purine-derived substance is a purine nucleoside selected from the group consisting of inosine, xanthosine, guanosine and adenosine.

16. The method according to claim 14, wherein the purine-derived substance is a purine nucleotide selected from the group consisting of inosinic acid, xanthylic acid, guanylic acid and adenylic acid.

17. A method for producing a purine nucleotide, which comprises
producing a purine nucleoside by the method according to claim 15,
reacting the purine nucleoside with a phosphate donor selected from the group consisting of polyphosphoric acid, phenyl phosphate and carbamyl phosphate, and a microorganism having an ability to produce a nucleoside-5'-phosphoric acid ester or acid phosphatase to produce a purine nucleotide, and
collecting the purine nucleotide.
